(19) Europäisches Patentamt
European Patent Office
Office européen des brevets

(11)  EP 2 488 866 B1

(12)  **EUROPEAN PATENT SPECIFICATION**

(45) Date of publication and mention
of the grant of the patent:
**29.04.2015 Bulletin 2015/18**

(51) Int Cl.:
*G01N 33/53* [(2006.01)]   *G01N 33/58* [(2006.01)]
*C12Q 1/25* [(2006.01)]

(21) Application number: **10822850.3**

(22) Date of filing: **14.10.2010**

(86) International application number:
**PCT/GB2010/001913**

(87) International publication number:
**WO 2011/045570 (21.04.2011 Gazette 2011/16)**

(54)  **ELISA SIGNAL AMPLIFCATION SYSTEM USING MAGNETIC BEAD MOVEMENT DETECTION.**

ELISA SIGNALVERSTÄRKUNG  MITTELS BEWEGUNG VON MAGNETISCHE KÜGELCHEN.

SYSTÈME D'AMPLIFCATION POUR ELISA UTILISANT LA DÉTECTION DU MOVEMENT D'UNE PARTICULE MAGNETIQUE.

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.10.2009 GB 0918016
14.10.2009 US 251562 P**

(43) Date of publication of application:
**22.08.2012 Bulletin 2012/34**

(73) Proprietors:
• **Centre National de la Recherche Scientifique
(CNRS)
75016 Paris (FR)**
• **University of Portsmouth
Higher Education Corporation
Portsmouth PO1 2UP (GB)**
• **École Normale Supérieure
75005 Paris (FR)**
• **Nanotecnologias
1000-029 Lisboa (PT)**

(72) Inventors:
• **BENSIMON, David
F-75005 Paris (FR)**
• **DE FREITAS, Paulo Jorge Peixeiro
P-1000-029 Lisboa (PT)**
• **FIRMAN, Keith
Portsmouth PO1 2DY (GB)**

(74) Representative: **Regimbeau
20, rue de Chazelles
75847 Paris Cedex 17 (FR)**

(56) References cited:
WO-A1-00/46357          WO-A1-00/71681
WO-A2-03/078966          US-A1- 2005 191 687

• KEJIK P ET AL: "An integrated micro-Hall probe
for scanning magnetic microscopy", SENSORS
AND ACTUATORS A, ELSEVIER SEQUOIA S.A.,
LAUSANNE, CH, vol. 129, no. 1-2, 24 May 2006
(2006-05-24), pages 212-215, XP025081800, ISSN:
0924-4247, DOI: DOI:10.1016/J.SNA.2005.11.061
[retrieved on 2006-05-24]
• TAMANAHA C R ET AL: "Magnetic labeling,
detection, and system integration",
BIOSENSORS AND BIOELECTRONICS,
ELSEVIER BV, NL, vol. 24, no. 1, 15 September
2008 (2008-09-15), pages 1-13, XP022820367,
ISSN: 0956-5663, DOI: DOI:10.1016/J.BIOS.
2008.02.009 [retrieved on 2008-02-16]
• Keir C Neuman ET AL: "Single-molecule force
spectroscopy: optical tweezers, magnetic
tweezers and atomic force microscopy", Nature
Methods, vol. 5, no. 6, 1 June 2008 (2008-06-01),
pages 491-505, XP055144881, ISSN: 1548-7091,
DOI: 10.1038/nmeth.1218
• STRICK T R ET AL: "BEHAVIOR OF
SUPERCOILED DNA", BIOPHYSICAL JOURNAL,
CELL PRESS, US, vol. 74, no. 4, 1 April 1998
(1998-04-01), pages 2016-2028, XP000905303,
ISSN: 0006-3495

**(Cont. next page)**

- **Iwijn De Vlaminck ET AL: "Recent Advances in Magnetic Tweezers", Annual Review of Biophysics, vol. 41, no. 1, 9 June 2012 (2012-06-09), pages 453-472, XP055144883, ISSN: 1936-122X, DOI: 10.1146/annurev-biophys-122311-100544**

SEQUENCE LISTING

<110> Le Centre National De La Recherche Scientifique university of Portsmouth Higher Education Corporation L'Ecole Normale Supérieure Instituto de Engenharia de Sistemas e Computadores Para os Microsistemas e Nanotecnologias

<120> Biosensor

<130> P524935PCT

<150> GB 0918016.7
<151> 2009-10-14

<150> US 61/251,562
<151> 2009-10-14

<160> 3

<170> PatentIn version 3.5

<210> 1
<211> 21
<212> DNA

<213> Artificial sequence

<220>
<223> Aptamer

<400> 1
ggtaggtgtg gttggtgcaa c        21

<210> 2
<211> 23
<212> DNA
<213> Artificial sequence

<220>
<223> Aptamer

<400> 2
ggtaggtttg gttggtgcaa cgc         23

<210> 3
<211> 23
<212> DNA
<213> Artificial sequence

<220>
<223> Aptamer

<400> 3
ggtaggtagg tatggtgcaa cgc         23

**Description**

The present invention relates to a detection system, uses thereof and methods relating thereto.

An enzyme-linked immunosorbent assay (ELISA) is a biochemical technique used mainly in immunology to detect the presence of an antibody or an antigen in a sample. ELISA has been used as a diagnostic tool in medicine and plant pathology, as well as a quality control check in various industries.

A particular type of ELISA, known as a "sandwich" ELISA, is used to detect the presence of a particular antigen in a sample. However, one problem with ELISA is that non-specific absorption of antibody, within the reaction vessel, can lead to false positive results. In addition, if the antigen is present in the sample at a very low level, the assay may not detect the presence of the antigen, even though it is contained in the sample, as the signal may not be sufficient to distinguish it from background signal. Therefore, the inventors for this application have developed an amplification system to amplify the signal from a sandwich ELISA.

The inventors have also developed a sensing apparatus which can be used, for example, to detect a signal from a sandwich ELISA. This is based on an optical magnetic tweezer system, which measures enzyme-based manipulation of DNA, at the single molecule level, by detecting movement of a magnetic bead which is used to stretch a surface tethered DNA molecule. Movement of the magnetic bead is detected through an optical system located below the tethered DNA. In the device described herein, the movement of the magnetic bead is detected using an electronic sensor, which in turn can be used to detect the signal from the ELISA. In a first aspect, an amplification system for amplifying a signal in a sandwich ELISA assay is provided, the system comprising:

a binding molecule releasably attached to a first releasing molecule; and
a plurality of signalling molecules immobilised on a substrate,
wherein, in use, the first releasing molecule is released from the binding molecule by a cleaving agent, and wherein, once released, the first releasing molecule causes a plurality of signalling molecules to be released from the substrate.

This amplification system allows amplification of a signal produced, for example, in an ELISA assay. In a normal sandwich ELISA assay, an antigen is captured on a surface by a capture antibody. A detecting antibody then binds to the antigen so that it is "sandwiched" by the capture antibody and the detecting antibody. Detection of the antigen is indicated by production of a signal. This signal is produced by a signal producing molecule, such as an enzyme, which is attached either to the detecting antibody or to a secondary antibody which binds to the detecting antibody. Either way, there is normally one enzyme molecule per antigen molecule to produce a signal. Obviously, if there is a low level of antigen in a sample, a low number of detecting antibodies will bind to the antigen, resulting in a low number of enzyme molecules being bound to the antigen (via the detecting antibody or secondary antibody) to produce a signal. This may mean that the signal that is produced is relatively weak due to the low number of enzymes. Therefore, the signal may be difficult to detect. For example, the relatively weak signal may be obscured by background noise.

In the present system, the binding molecule releasably attached to a first releasing molecule is comparable to either the detecting antibody attached to an enzyme or the secondary antibody attached to an enzyme in a normal sandwich ELISA. However, instead of producing a signal, the binding molecule releasably attached to a first releasing molecule functions to amplify the production of a signal.

In a particular embodiment, the binding molecule releasably attached to a first releasing molecule binds to an antigen which has already been immobilised on a surface, for example, by a capture antibody. The first releasing molecule is then caused to be release from the binding molecule by the cleaving agent. When the first releasing molecule, which is attached to the binding molecule, is released, it causes a plurality of signalling molecules (e.g. an enzyme) to be released each of which can contribute to the production of a signal. Since a plurality of signalling molecules are released per antigen molecule, this has the effect of amplifying the signal that is produced. This is in contrast to the one-to-one antigen to signalling molecule relationship in a normal ELISA assay. If one first releasing molecule causes thirty signalling molecules to be released, this causes a thirty-fold increase in the number of signalling molecules producing a signal. This will produce a stronger signal which will be much easier to detect. Therefore, the present system allows a relatively low level of antigen to be detected by increasing the signal-to-noise ratio so that a positive result is obtained even with relatively low levels of antigen.

Further, due to the separate amplification step in which the first releasing molecule causes a plurality of signalling molecules to be released, the system provides a reduction in background signalling due to non-specific binding of the binding molecule to surfaces within the reaction chamber. The first-stage release of the first releasing molecule being released from the binding molecule occurs in a separate reaction chamber from the second-stage release of signalling molecules being released from the substrate, thus reducing background noise.

The first releasing molecule can be attached to the binding molecule in any suitable way so that it can be released from the binding molecule in response to the cleaving agent, i.e. the cleaving agent causes the first releasing molecule to detach from the binding molecule. The first releasing molecule may be attached directly to the binding molecule. In this case, the cleaving agent may cause the first releasing molecule to detach from the binding molecule by causing cleavage of the binding molecule so that the first releasing molecule is released with a portion of the binding molecule still attached thereto. Alternatively, the first releasing molecule may be caused to be cleaved so that a functional portion of the first releasing molecule is released. In any event, it is necessary to have a suitable cleavage site on which the cleaving agent acts in order to allow the first releasing molecule to be detached from the binding molecule.

In one embodiment, the first releasing molecule is releasably attached to the binding molecule via a linker. A suitable linker would be apparent to one skilled in the art. Preferably, the cleaving agent causes cleavage of the linker to release the first releasing molecule from the binding molecule, i.e. the linker has a cleavage site which is acted upon by the cleaving agent.

The attachment of the binding molecule to the first releasing molecule, whether it is via a linker or not, can be done in any suitable way and appropriate attachments and methods will be readily apparent to those skilled in the art. The attachment can be through covalent conjugation or through a strong non-covalent interaction. For example, the attachment of the two molecules may be achieved by a biotin-streptavidin linkage. Preferably, such a linkage also contains a cleavage site for the first releasing molecule. The nature of the attachment, or linker, between the binding molecule and the first releasing molecule will depend to a certain extent on the identity of the binding molecule and/or the first releasing molecule; for example, whether the binding molecule and first releasing molecule are both proteins or both polynucleotides.

As indicated above, the binding molecule releasably attached to the first releasing molecule should have a cleavage site on which the cleaving agent acts in order to cause at least a functional portion of the first releasing molecule to be released from the binding molecule. Preferably, the whole of the first releasing molecule is released from the binding molecule. If the binding molecule is attached to the first releasing molecule via a linker, the cleavage site is preferably in the linker. The cleavage site can be any suitable cleavage site which can be recognised and acted upon by the cleaving agent to cause the first releasing molecule to detach from the binding molecule and will to a certain extent depend on the nature of the cleaving agent.

The cleaving agent can be any suitable cleaving agent which causes cleavage of the first releasing molecule from the binding molecule. This will take place at a cleavage site. Effectively, the cleaving agent causes a covalent bond to break in the molecule made up of the binding molecule attached to the first releasing molecule. The chemical bond that is broken should be a specific bond rather than a random bond in the molecule. Suitable cleaving agents will be apparent to those skilled in the art. For example, the cleaving agent may be electromagnetic radiation, an agent capable of chemical cleavage (e.g. a reducing agent capable of chemical cleavage), or an enzyme capable of cleavage.

If the cleaving agent is electromagnetic radiation, a chemical bond in the molecule can be cleaved in the process of photolysis. The electromagnetic radiation is preferably of a particular wavelength to cause cleavage of a specific bond in the molecule made up of the binding molecule attached to the first releasing molecule. In one embodiment, the bond that is cleaved is contained in a photo-cleavable linker. Suitable bonds which are cleavable by photolysis and suitable photo-cleavable linkers are well known to those skilled in the art. For example, see Pandori MW et al "Photochemical control of the infectivity of adenoviral vectors using a novel photocleavable biotinylation reagent." Chem. Biol. 2002 ay;9(5):567-73 and Petrotchenko EV et al. "BiPS, a photocleavable, isotopically coded, fluorescent cross-linker for structural proteomics." Mol. Cell Proteomics. 2009 Feb;8(2):273-86.

If the cleaving agent is a reducing agent, a particular chemical bond in the molecule can be reduced so that it is cleaved to release the first releasing molecule from the binding molecule. The reducing agent should cause cleavage of a specific bond. For example, if the first releasing molecule is attached to the binding molecule via a disulphide bond, a reducing agent can be used to reduce the disulphide bond so that it breaks, thereby releasing the first releasing molecule from the binding molecule. Suitable reducing agents are well known to those skilled in the art.

If the cleaving agent is an enzyme, it should be capable of cleaving a particular bond by recognising the cleavage site. Suitable enzymes are, for example, a protease, a restriction enzyme. Other suitable enzymes would be well known to those skilled in the art.

In a particular embodiment, the cleaving agent is a second releasing molecule. The second releasing molecule can be any suitable molecule which can cause the first releasing molecule to detach from the binding molecule. Preferably, the second releasing molecule is a reducing agent or an enzyme. In one embodiment, the second releasing agent is an enzyme. The identity of the second releasing molecule will depend on the nature of the binding molecule and/or the first releasing molecule. For example, if one or both of the binding molecule and the first releasing molecule are proteins, the second releasing molecule may be a molecule which is capable of cleaving proteins. The second releasing molecule may be a protease. Alternatively, the second releasing molecule may be a reducing agent if the binding molecule and first releasing molecule are attached via a disulphide bond. The second releasing molecule must release the first releasing molecule from the binding molecule so that the first releasing protein is functional. If one or both of the binding molecule and the first releasing molecule are polynucleotides, the second releasing molecule may be a molecule which is capable of cleaving polynucleotides. The second releasing molecule may be a restriction enzyme.

The first releasing molecule can be attached to the binding molecule in any suitable way so that the second releasing molecule is able to cause the first releasing molecule to detach from the binding molecule. The second releasing molecule should recognise a cleavage site at which it acts to cause the first releasing molecule to be detached from the binding molecule.

In one embodiment, the first releasing molecule is attached to the binding molecule via a polypeptide linker. This polypeptide linker can be cleaved, for example, by a protease which recognises a cleavage site in the polypeptide. Alternatively, the binding molecule may be attached to the first releasing molecule via a polynucleotide linker. This may be cleaved, for example, by a restriction enzyme with a restriction (cleavage) site in the polynucleotide linker. Suitable restriction enzymes are well known to those skilled in the art and their corresponding cleavage sites.

Preferably, there are a plurality of first releasing molecules which are releasably attached to the binding molecule so that each first releasing molecule can be detached from the binding molecule by the cleaving agent. Each of these may be attached via a linker. The plurality of first releasing molecules should all be the same releasing molecule. Having a plurality of first releasing molecules attached to the binding molecule adds to the level of amplification that takes place in the system.

The binding molecule may be any suitable binding molecule which is suitable for use in an ELISA assay to bind to an antigen of interest or to bind to an antibody of interest. Preferably, the binding molecule binds the antigen or antibody of interest specifically. The binding molecule may be an antibody, an aptamer, an HC molecule or a T- cell receptor. Preferably, the binding molecule is an antibody or an aptamer and, more preferably, the binding molecule is an antibody.

The term "antibody" is well known in the art. Herein it means an immunoglobulin or any functional fragment thereof. It encompasses any polypeptide that has an antigen-binding site. It includes but is not limited to monoclonal, polyclonal, monospecific, polyspecific, non-specific, humanized, human, single-chain, chimeric, synthetic, recombinant, hybrid, mutated, grafted, and in vitro generated antibodies. The term "antibody" encompasses antibody fragments such as Fab, F (ab') 2, Fv, scFv, Fd, dAb, and any other antibody fragments that retain antigen-binding function. Typically, such fragments would comprise an antigen-binding domain.

The term "aptamer" is well known in the art and includes nucleic acid aptamers (e.g. DNA, RNA and PNA), peptide aptamers and functional fragments thereof. A skilled person would be well aware of how to produce an aptamer, for example, as described in Ellington AD, Szostak JW (Aug 1990). "In vitro selection of RNA molecules that bind specific ligands". Nature 346 (6287): 818-22; Bock LC, Griffin LC, Latham JA, Vermaas EH, Toole JJ (Feb 1992). "Selection of single-stranded DNA molecules that bind and inhibit human thrombin". Nature 355 (6360): 564-6; and Hoppe-Seyler F, Butz (2000). "Peptide aptamers: powerful new tools for molecular medicine". J Mol Med. 78 (8): 426-30.

The term "MHC molecule" is well known to those skilled in the art and includes functional fragments thereof. The MHC molecule should be capable of binding to an antigen or antibody of interest. The MHC molecule may be a class I or class II MHC molecule.

The term "T-cell receptor" is well known to those skilled in the art and includes functional fragments thereof. The T-cell receptor should be capable of binding to an antigen or antibody of interest.

The identity of the binding molecule does not affect the present amplification system as long as it allows the amplification system to be used to amplify the signal in a sandwich ELISA assay.

Normally, in a sandwich ELISA, an antigen that is to be detected is bound to antibodies which are specific to that antigen and which are immobilised on a substrate. Another detecting antibody which is specific to that antigen also binds to the antigen to allow its presence to be detected. This forms an antigen which is in a 'sandwich' between two antibody molecules. In the present system, the binding molecule can act in the same way as the detecting antibody to allow detection of the antigen, i.e. the binding molecule may bind to an antigen that is to be detected using an ELISA assay. The signal can then be amplified and detected using the amplification system. Alternatively, the binding molecule may be used as a secondary binding molecule in an ELISA system. In this way, the binding molecule which forms part of the binding molecule-first releasing molecule conjugate recognises and binds to the detecting antibody which is bound to the antigen. The signal can then be amplified using the amplification system. The advantage of the binding molecule being used as a secondary binding molecule is that it has much broader applicability, for example, if it can specifically bind to any mouse antibody rather than a particular antigen.

The signalling molecule can be any suitable molecule which is capable of producing a signal. The signalling molecule may be capable of producing a signal directly, for example, green fluorescent protein (GFP) which fluoresces when exposed to blue light. Alternatively, the signalling molecule may produce a signal indirectly, for example, horseradish peroxidase or luciferase which can catalyse the breakdown of chemiluminescent substrates to produce light. Many suitable signalling molecules are well known to those skilled in the art and include GFP, EGFP, Lux, horseradish peroxidase and luciferase. In one embodiment, the signalling molecule is a manipulating agent such as a molecular motor. Preferably, the signalling molecule is a protein.

The signalling molecule is immobilised on a substrate. Suitable ways of immobilising a signalling molecule are well known to those skilled in the art. Further, suitable substrates are also well known to those skilled in the art. For example, the substrate may be a surface formed of gold, glass, a plastics material or a covalently cross linked ally] dextrose gel such as sephacryl® beads. In one embodiment, the substrate can be a polystyrene surface, such as a polystyrene multiwell plate. Alternatively, the substrate may be a dipstick or membrane.

The signalling molecule may be attached to an immobilising molecule which can be immobilised on a substrate. Suitable immobilising molecules are well known to those skilled in the art. For example, biotin can be conjugated to the signalling molecule which will allow it to be immobilised on an avidin-coated surface, preferably a streptavidin-coated surface, since biotin binds to avidin relatively strongly. The signalling molecule may be attached to the immobilising molecule via a linker. The linker may contain a cleavage site which can be recognised by the first releasing molecule so that the signalling molecule can be cleaved and released from the substrate. For example, if the linker is a polypeptide, it may contain a cleavage site for a protease.

The signalling molecule preferably contains a cleavage site which is recognised and cleaved by the first releasing molecule to release the signalling molecule from the substrate. The cleavage site can be anywhere in the signalling molecule as long as a functional portion of the signalling molecule is released from the substrate so that it can produce a signal. If the signalling molecule is attached to an immobilising molecule, the cleavage site may be contained in the immobilising molecule so that when the first releasing molecule causes the signalling molecule to be released from the substrate, a portion of the immobilising molecule may still be attached to the signalling molecule.

The first releasing molecule can be any suitable molecule which can cause the signalling molecule to be detached from a substrate. Preferably, the first releasing molecule is an enzyme. The enzyme should be capable cleaving the signalling molecule from the substrate. The identity of the first releasing molecule will depend on the nature of the signalling molecule and/or the way in which it is immobilised on a substrate. For example, the first releasing molecule may be a protease to cleave, for example, a polypeptide linker. Alternatively, it may be a restriction enzyme to cleave, for example, a polynucleotide linker. In another embodiment, it may be a reducing agent to cleave, for example, a disulphide bond.

The specificity of the first releasing molecule and the second releasing molecule, where one is used, should not be the same so that the second releasing molecule does not cause the signalling molecule to be released from the substrate instead of the first releasing providing this function. If the second releasing molecule caused the signalling molecule to be released from the substrate, the system could provide a false positive result.

In a particular embodiment, a column-based system may be used for the assay, which relies upon the use of either Protein A, G or A/G based sepharose columns for immobilisation of the Fc fragment of antibodies (as capture antibodies) to be utilised in the assay (Choice of A, G or A/G is dependent on the source of antibodies). Since only the Fc portion of the antibody is bound, the Fab portion is free to bind to the target (i.e. an antigen) and therefore the sandwich can be attained with the use of a binding molecule of the present disclosure. Alternative systems could be used for the immobilisation of biotinylated aptamers (streptavidin sepharose/agarose etc). With this system it would be possible to scale the column type and also media volume in order to obtain a response.

The amplification system can be used with existing ELISA, where the binding molecule is equivalent to, for example, the secondary antibody in a standard sandwich ELISA reaction within a multiwell plate. The cleaving agent is provided after the normal washes and will release the first releasing molecule - non-specifically bound materials remain in the multiwell plate. The non-bound contents of the multiwell plate (which includes the released first releasing molecule where antigen is present) can be passed, for example, over a column matrix of immobilised signalling enzyme (e.g. a spin-column system), allowing release of the signalling molecule for detection using an appropriate method known to those skilled in the art.

The present disclosure also provides the use of the amplification system described above to amplify the signal in an ELISA assay.

Furthermore, an amplification method for amplifying a signal in a sandwich ELISA assay is provided, the method comprising:

exposing a cleaving agent to a first releasing molecule, which is releasably attached to a binding molecule, to release the first releasing molecule from the binding molecule; and
exposing the released first releasing molecule to a plurality of signalling molecules, which have been immobilised on a substrate, to release the plurality of signalling molecules from the substrate.

Preferable features of the method are as described above with respect to the amplification system.

The method should be carried out when the binding molecule is bound to an antigen or a primary antibody (detecting antibody) which is bound to an antigen. In one embodiment, the method further comprises the initial step of exposing the binding molecule, which is releasably attached to the second releasing molecule, to an antigen or an antibody bound to an antigen.

The method may also further comprise detecting a signal produced by the released signalling molecules. Suitable ways in which the signal may be detected are well known to those skilled in the art. For example, a chemiluminescent substrate may be broken down by the released signalling molecule to produce light which can then be detected.

In this respect, the signalling molecule may be horseradish peroxidase or luciferase. Light produced from a chemiluminescent substrate may be measured using a photographic film, a luminometer, a scintillation counter or an ELISA plate reader. Light at a specific wavelength may be measured. Alternatively, the signalling molecule may fluoresce upon exposure to light of a particular wavelength. This can be measured using a fluorometer.

In one embodiment, the above method works in the following way. A capture antibody is immobilised on a substrate in a reaction chamber. An antigen recognised by the capture antibody binds to the capture antibody. A binding molecule which is releasably attached to a first releasing molecule is introduced into the reaction chamber so that the binding molecule binds to the antigen which is immobilised on the substrate via the capture antibody. The reaction chamber is then washed so as to remove any unbound binding molecules and any other unbound material. A cleaving agent, such as a second releasing molecule, is introduced into the reaction chamber so that the first releasing molecule is detached from the binding molecule, i.e. the first releasing molecule is free in solution. The solution in the reaction chamber is then removed and introduced into a second reaction chamber in which signalling molecules are immobilised on a substrate. The first releasing molecule which is in solution causes a plurality of the signalling molecules to detach from the substrate so that they are free in solution. The solution, containing the signalling molecules, is then removed from the second reaction chamber and this solution is tested for the presence of the signalling molecules in a way which is suitable for the particular signalling molecule.

A kit for amplifying a signal in an ELISA assay is also provided, the kit comprising: a binding molecule releasably attached to a first releasing molecule; and
a plurality of signalling molecules which can be immobilised on a substrate.

In one embodiment, the kit further comprises a second releasing molecule.

The kit may also comprise reagents for use with the signalling molecule to allow detection of the signal.

In one embodiment, the plurality of signalling molecules are immobilised on a substrate.

In another embodiment, the signalling molecule is a manipulating agent such as a molecular motor.

In a second aspect, the disclosure provides a sensing apparatus, the apparatus comprising:

a surface;
an elongate molecule attached at one end to the surface;
a magnetic bead attached to the other end of the elongate molecule;
an element to produce a magnetic field; and
an electronic sensor to detect movement of the magnetic bead,
wherein the element produces a magnetic field which exerts a force on the magnetic bead to stretch the elongate molecule into an extended form, and wherein the electronic sensor detects movement of the magnetic bead resulting from movement of the elongate molecule.

The sensing apparatus can be used to detect any signal which is capable of causing the elongate molecule to move. This signal may be, for example, a manipulating agent such as a molecular motor, an energy source or UV light, as discussed in more detail below.

In one embodiment, the sensing apparatus can be used to detect the presence of a manipulating agent which is capable of causing the elongate molecule to move. The manipulating agent can be any agent which can interact with the elongate molecule so that the elongate molecule moves in some way. The exact nature of the manipulating agent will depend to a degree on the nature of the elongating molecule.

In one embodiment, the manipulating agent is a molecular motor which is capable of causing the elongate molecule to move. When a molecular motor is present, the molecular motor can bind to the elongate molecule and cause it to move. This has the effect of causing the magnetic bead to move and the electronic sensor detects this movement of the magnetic bead.

PCT application WO 00/71681 discloses a complex of a molecular motor bound to a nucleic acid sequence, wherein the said nucleic acid can be bound to a magnetic bead, causing the bead to move during translocation of the nucleic acid.

The molecular motor can be any suitable molecule which is capable of causing movement of the elongate molecule. Movement of the elongate molecule can be through contraction, stretching, rotation and/or supercoiling. This, in turn, causes movement of the magnetic bead so that it is displaced and/or rotated. In one embodiment, the elongate molecule is caused to contract and, optionally, rotate. Suitable molecular motors include topoisomerases, polymerases (such as DNA polymerase), helicases, recombinases, chromatin and chromatin-remodelling factors, regulation factors, translocases and restriction-modification enzymes. For example, a DNA translocase such as Fts could be used or a type I restriction-modification enzyme such as EcoR.1241. Other suitable enzymes are Rad54, Topoisomerase 1 , UvrD, RecQ and gp41. Preferably, the molecular motor is a DNA manipulating enzyme.

In one embodiment, the sensing apparatus further comprises a molecular motor bound to the elongate molecule to cause movement of the elongate molecule. When the sensing apparatus comprises a molecular motor, it can be used to detect the release or production of an energy source for the molecular motor. In this embodiment, the sensing apparatus is used to detect the energy source via the activity of the molecular motor. In order for the molecular motor to cause movement of the elongate molecule, it requires an energy source to drive the movement of the elongate molecule. Therefore, when an energy source is released or produced, the molecular motor uses this energy source to drive the movement of the elongate molecule. This causes the magnetic bead to move which is detected by the electronic sensor. When the energy source is used up and is no longer being released or produced, the molecular motor no longer has an energy source to cause movement of the elongate molecule. Therefore, the elongate molecule may return to an extended form (because of the force exerted on the magnetic bead attached to the elongate molecule) or may cease movement. When the elongate molecule has been caused to contract, the magnetic field which exerts a force on the magnetic bead causes the elongate molecule to extend into an elongate form. This extension causes the magnetic bead to move which can be detected by the electronic sensor. Alternatively, when the elongate molecule has been caused to rotate, this rotation will stop when the energy source has been used up. When the elongate molecule has been caused to contract and rotate by the molecular motor, the elongate molecule will be extended by the magnetic field and may also rotate when the energy source is used up.

The energy source can be any suitable energy source which the molecular motor can utilise to cause movement of the elongate molecule. For example, the energy source may be a nucleotide triphosphate, such as ATP.

Preferably, the elongate molecule should allow the sensing apparatus to work in a reversible manner, i.e. allow the sensing apparatus to function repeatedly by allowing and measuring multiple movements of the elongate molecule. For example, where a molecular motor is bound to the elongate molecule, the presence of a suitable energy source causes movement of the elongate molecule via the molecular motor. Once the energy source is used up, the molecular motor stops, causing the elongate molecule to stop moving or allowing it to return to its extended form (depending on the nature of the movement). Once the energy source returns, the molecular motor will again cause the elongate molecule to move. Therefore, in such an embodiment, the sensing apparatus can act in a repeatable manner.

The surface may be any suitable surface to which the elongate molecule can be attached. Suitable surfaces are well known to those skilled in the art. For example, the surface may be glass, gold (e.g. gold-pads), or a plastics material, such as polystyrene.

The elongate molecule can be attached to the surface in any suitable way and such ways are well known to those skilled in the art. For example, a biotin molecule can be conjugated to one end of the elongate molecule. This allows the elongate molecule to be immobilised on an avidin-coated surface, preferably a streptavidin-coated surface, via the biotin molecule. Alternatively, the elongate molecule may be attached to the surface by conjugating a hapten such as digoxigenin (DIG) to the elongate molecule. An anti-hapten antibody such as antiDIG is coated on the surface so that the antibody binds to the hapten conjugated to the end of the elongate molecule. One way in which the elongate molecule may be attached to a gold surface is through chemical attachment where the elongate molecule is thioylated.

The elongate molecule can be any suitable molecule as long as it can be caused to move by the signal that is to be detected. For example, where a manipulating agent, e.g. a molecular motor, is the signal, the manipulating agent causes the elongate molecule to move. The elongate molecule is preferably formed from a single continuous chain of molecules so that it has a long string-like structure. For example, a polypeptide chain or a polynucleotide chain, when in an extended conformation, would have an elongate shape and be elongate molecules. The elongate molecule may be between about 500 nm and about 5 $\mu$m in length. Preferably, the elongate molecule is between about 500 nm and about 3 $\mu$m in length. This is the length of the longest dimension of the elongate molecule. Preferably, the elongate molecule is a polynucleotide. In one embodiment, the elongate molecule is a DNA molecule. Where the elongate molecule is a polynucleotide, preferably, there are no other molecules, e.g. proteins, attached to it.

In alternative embodiments, the elongate molecule may have other molecules bound to it. For example, if the elongate molecule is a polynucleotide, it may have two proteins bound to it at different points of the elongate molecule, wherein the two proteins bind to each other so that there is a loop in the elongate molecule. In such an embodiment, the manipulating agent may be an agent which disrupts protein interactions, for example, a surfactant. The manipulating agent disrupts the interaction between the two proteins so that they are no longer bound to each other, thereby causing the elongate molecule to move as the loop that was contained in the elongate molecule extends into a fully extended conformation due to the force being exerted on it by the magnetic field on the magnetic bead. In this way, the sensing apparatus can be used to study protein-protein interactions.

In yet another embodiment, the elongate molecule may be in a constrained conformation so that once it is acted upon by the signal, the constraint on the structure of the elongate molecule is released causing it to extend due to the force being exerted on it by the magnetic field on the magnetic bead. For example, the elongate molecule may be DNA which takes the form of a helix. The signal, in the form of UV light, may cause a break in one of the DNA strands by breaking a bond. Alternatively, a manipulating agent such as a restriction 'nicking' enzyme may cause a break or nick in one of the D A strands. When one of the strands of the DNA contains a break or nick, the torsional strain in the molecule is released allowing the DNA to untwist, thereby extending and causing movement of the magnetic bead.

As will be appreciated from the embodiments of the elongate molecule described above, when the elongate molecule is stretched into "an extended form" by the force of the magnetic field on the magnetic bead, this does not mean that the elongate molecule cannot extend further after being acted upon by the signal such as a manipulating agent. For example, the elongate molecule may have a loop contained therein or may be in a constrained configuration which, when released, allows the elongate molecule to extend. The term "stretched into an extended form" means that the elongate molecule is stretched into an extended form given the structural constraints of that particular molecule.

The elongate molecule has a magnetic bead attached at its opposite end relative to the attachment to the surface. This is the opposite end of the elongate structure of the molecule so that the molecule can form a long string-like structure between the bead at one end and the surface at the other. The elongate molecule can be attached to the magnetic bead in any suitable way and such ways are well known to those skilled in the art. For example, a biotin molecule can be conjugated to the end of the elongate molecule. The surface of the magnetic bead can be coated with streptavidin so that the biotin molecule on the elongate molecule can be attached to the bead. Alternatively, a hapten-antibody attachment could be used (e.g. DIG-antiDIG), as discussed above.

The magnetic bead can be any suitable bead so that it can be attached to the elongate molecule. For example, the magnetic bead may be a latex bead with ferrites incorporated therein. The magnetic bead may be a ferromagnetic bead. Alternatively, the magnetic bead may be a paramagnetic bead and, preferably, a superparamagnetic bead. The magnetic bead may be between about 200 nm and about 5 $\mu$m in diameter. Preferably, the magnetic bead is between about 200 nm and about 2 $\mu$m in diameter. More preferably, the magnetic bead is between about 500 nm and about 1.5 $\mu$m in diameter and, most preferably, the magnetic bead is about 1 $\mu$m in diameter.

The element to produce a magnetic field can be any suitable element as long as it is able to exert a force on the magnetic bead to stretch the elongate molecule into an extended form. Preferably, the elongate molecule is positioned in the sensing apparatus so that it is orientated substantially vertically in use. Preferably, the magnetic bead is positioned at the uppermost end of the elongate molecule in use. Preferably, the magnetic field exerts a substantially vertical upward force on the magnetic bead in use to stretch the elongate molecule into an extended form. The element which produces a magnetic field may be a ferromagnet. Alternatively, the element may be an electromagnet, i.e. a wire carrying an electrical current. When an electric current flows through a wire, a magnetic field is produced. This can be used to exert a force on another magnetic object. In order to increase the magnetic field that is produced by the wire, it may be wound into a coil. Further, the electromagnet may or may not have a core of ferromagnetic material. In one embodiment, the element is a ring of wire, the ring being formed from a single piece of wire. Alternatively, the element may be a coil of wire formed from a plurality of turns of wire. As will be appreciated by one skilled in the art, the magnetic field produced by the element will depend on the current flowing through the wire and the wire forming the electromagnet, e.g. the number of turns in the coil. The magnetic field must be strong enough so that it can exert a force on the magnetic bead to stretch the elongate molecule into an extended form. However, the force should not be so strong that the elongate molecule cannot be caused to contract due to an excessive force being applied to the magnetic bead. In one embodiment, a force of between about 0.1 pN to about 100 pN is appropriate to cause the required levitation of the magnetic bead and to allow contraction of the elongate molecule. More preferably, the force is between about 0.1 pN to about 50 pN and, more preferably still, between about 0.1 pN to about 20 pN. The vertical component of the magnetic field generated by the element, at the level of the bead, is between about 0.1 and about 5 mT. These forces and field strengths are preferably used for a paramagnetic bead of about 1 $\mu$m in diameter.

The electronic sensor can be any sensor that is suitable for detecting the movement of the magnetic bead. The bead moves as a result of the elongate molecule moving, for example, by contracting, extending, rotating or supercoiling. In one embodiment, the movement of the magnetic bead that is detected by the sensor is movement of the magnetic bead parallel to the long axis of the elongate molecule when in its extended form (typically movement of the bead along a vertical axis when the sensing apparatus is in use). In one embodiment, when the elongate molecule is a polynucleotide, the polynucleotide, and therefore the magnetic bead, may rotate as the polynucleotide contracts in length. Therefore, in order to measure the contraction of the polynucleotide, the electronic sensor could measure the rotation of the magnetic bead which would be indicative of the contraction of the polynucleotide. Preferably, the electronic sensor is a magnetoresistive sensor or a hall effect sensor.

A particular type of magnetoresistive sensor is a TMR (tunnel magnetic resistance) device. This is a device in which a very thin insulating layer is inserted between two ferromagnetic layers. A TMR device uses the phenomenon that the tunneling current flowing through the insulating layer is changed by the relative angle of magnetization of each metal element. Such a field is subject to external effects from external magnetic fields. For example, see United States Patent 6528326 or US Application no. 20090135526.

Another type of magnetoresistive sensor is a GMR (giant magnetic resistance) device. The giant magnetoresistive effect is a quantum mechanical effect, due to electron spin, in which a significant reduction of electrical resistance is produced in a thin-layer structure of alternating magnetic and non-magnetic layers in the presence of an external magnetic field. See Hinchey εt Mills. "Magnetic properties of superlattices formed from ferromagnetic and antiferromagnetic materials." Phys. Rev. B 33, 3329 - 3343 (1986).

The Hall effect is the production of a voltage difference (the Hall voltage) across an electrical conductor, transverse to an electric current in the conductor and a magnetic field perpendicular to the current. For example, see: Leong Z.Y. et al. Journal of Magnetism and Magnetic Materials. Vol. 310, Issue 2, Part 3, March 2007, pages e635-e637.

The sensing apparatus may further comprise a component to magnetise the magnetic bead. When the magnetic bead is a paramagnetic bead, it may be advantageous to have a component which creates a magnetic field which induces a magnetic moment in the bead. This allows easier detection of any movement of the bead. The vertical component of the magnetic field generated by the component, at the level of the bead, is between about 0.1 and about 5 mT. This field strength is preferably used for a paramagnetic bead of about 1 $\mu$m in diameter. Preferably, the magnetic field produced by the component does not interfere with the magnetic field produced by the element and thus does not substantially affect the way in which the magnetic field produced by the element exerts a force on the magnetic bead. This can be done, for example, using a direct current in the element to produce the magnetic field and an alternating current in the component to magnetise the magnetic bead. Alternatively, the element which produces the magnetic field can be used to magnetise the bead and to provide a force on the bead.

In one embodiment, any electrical parts of the sensing apparatus are passivated so that the apparatus can be used in physiological conditions. If any electrical parts are not passivated, sodium ions are liable to penetrate into oxide layers. Any electrical parts can be passivated, for example, by incorporating an aluminium nitride layer to resist sodium ion penetration or by coating surfaces with casein, BSA or another neutral protein.

In another embodiment, the sensing apparatus is contained in a chamber which allows the apparatus to function in a stable environment. For example, the apparatus may be integrated into a microfluidic system such as a PDMS chamber to improve the stability and reliability of the apparatus.

The present disclosure also provides the use of the sensing apparatus described above for detecting the release or production of a molecular motor or an energy source.

The present invention provides a detection system for detecting a signal in a sandwich ELISA assay, the detection system comprising an amplification system and a sensing apparatus, the amplification system comprising:

a binding molecule releasably attached to a first releasing molecule; and
a plurality of manipulating agents immobilised on a substrate,
and the sensing apparatus comprising:

a substrate;
an elongate molecule attached at one end to the substrate;
a magnetic bead attached to the other end of the elongate molecule;
an element to produce a magnetic field; and
a sensor to detect movement of the magnetic bead,
wherein the element produces a magnetic field which exerts a force on the magnetic bead to stretch the elongate molecule into an extended form, and wherein the sensor detects movement of the magnetic bead resulting from movement of the elongate molecule,
wherein, in use, the first releasing molecule is released from the binding molecule by a cleaving agent, wherein, once released, the first releasing molecule causes a plurality of manipulating agents to be released from the substrate, wherein, once released, the manipulating agents interact with the elongate molecule in the sensing apparatus and cause movement of the elongate molecule, and wherein the movement of the elongate molecule causes movement of the magnetic bead which is detected by the sensor.

The detection system described above is a combination of the amplification system described above and the detection apparatus described above. The initial amplification part of the detection system is the same as the amplification system of the first aspect of the disclosure wherein the signalling molecule is a manipulating agent. The release of the manipulating agent is detected using the sensing apparatus of the second aspect of the disclosure. Accordingly, all the definitions and description of the features of the first aspect and second aspect of the disclosure are equally applicable to the detection system of the invention.

Preferably, the manipulating agent is a molecular motor which binds to the elongate molecule in the sensing apparatus to cause movement of the elongate molecule.

The present invention also provides the use of the detecting system described above for amplifying and detecting a signal.

Furthermore, the present invention provides a detection method for detecting a signal in a sandwich ELISA assay, the method comprising:

exposing a cleaving agent to a first releasing molecule, which is releasably attached to a binding molecule, to release the first releasing molecule from the binding molecule;
exposing the released first releasing molecule to a plurality of manipulating agents which have been immobilised on a substrate to release a plurality of manipulating agents from the substrate; and
exposing the released manipulating agents to a sensing apparatus to detect the signal,
wherein the sensing apparatus comprises:

a surface;
an elongate molecule attached at one end to the surface;
a magnetic bead attached to the other end of the elongate molecule;
an element to produce a magnetic field; and
an electronic sensor to detect movement of the magnetic bead,
wherein the element produces a magnetic field which exerts a force on the magnetic bead to stretch the elongate molecule into an extended form, wherein the electronic sensor detects movement of the magnetic bead resulting from movement of the elongate molecule and wherein the molecular motor causes movement of the elongate molecule which causes the magnetic bead to move, this movement being detected by the electronic sensor.

The features of the amplification method and any preferred or additional features are equally applicable to this initial amplification stage of the above method.

The present invention will now be described in detail, by way of example only, with reference to the figures in which:

Figure 1 shows a cartoon of a biological molecular amplifier:

Stage 1) Recognition of an antigen X in an ELISA sandwich assay with a protease B coupled to a streptavidin;
Stage 2) Release of the protease B by proteolysis of its bond (with protease A); and
Stage 3) Release by protease B of Lux.

Figure 2 shows an SDS-PAGE gel of the cleavage of GST-( PreScission™)-HsdR fusion protein by PreScission™ Protease. Increasing amounts (0, 0.01 , 0.05, 0.1 , 0.5, 1 , 2.5, 5, 7.5, 10 Units) of PreScission™ Protease were incubated with 100 ng (69 pM) concentrations of the fusion protein GST-(PreScission™)-HsdR. Proteolysis at the PreScission™ Protease cleavage site between the GST and HsdR fusion releases free HsdR and GST. Added PreScission™ Protease becomes visible above 1 Unit.

Figure 3 is a graph of %-release of GST following Prescission™ protease cleavage.

Figure 4 shows the surface attachment of the thrombin aptamer. Binding of the biotinylated aptamer to the streptavidin coated SPR chip (Biacore) indicating complete binding of the aptamer to the surface.

Figure 5 shows SPR determination of thrombin-aptamer binding and dissociation. Increasing concentrations of thrombin was passed over the SPR chip with the aptamer bound to the chip surface as described in Figure 4. Thrombin concentration increases from 0 nM, 5 nM, 10 nM, 50 nM, 100 nM, 200 nM, 400 nM, 600 nM, 800 nM, 1000 nM.

Figure 6 shows a molecular dynamo device.

Figure 7 shows a magnetoresistive magnetic tweezer system.

Figure 8 is a schematic of a device and an optical picture of process chip (without microfluidic system integrated) showing the transparent propriety.

Figure 9 is a schematic of the bead magnetization, with (LEFT) and without (RIGHT) magnetic tweezers.

Figure 10 shows magnetic tweezers design.

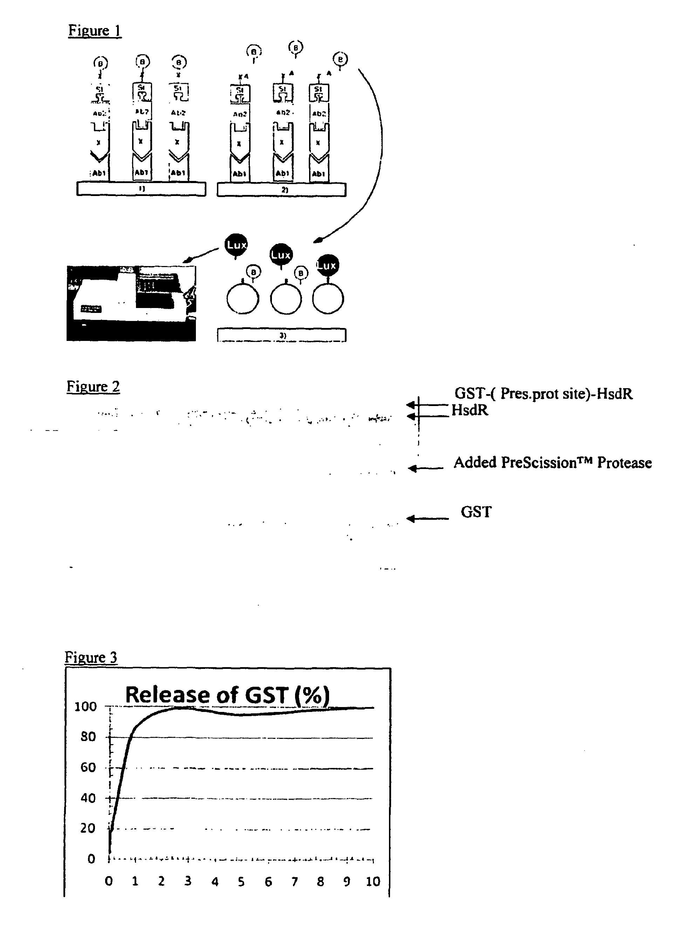

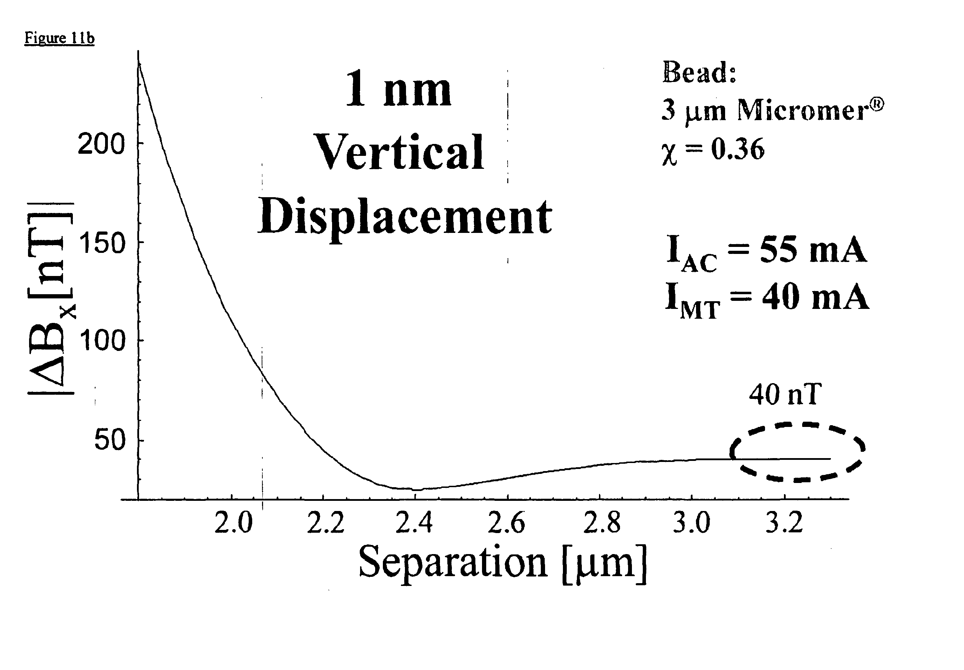

Figure 1 1 shows theoretical values of the fringe field expected from a magnetic bead in the platform proposed. At a distance of 3 $\mu$m above the sensor, one bead with 3 $\mu$m of diameter creates a fringe field at the sensor level of about 50 uT (Figure 1 1a), therefore, a variation in the vertical position of 1 nm implies a field change of 40 nT (Figure 11b).

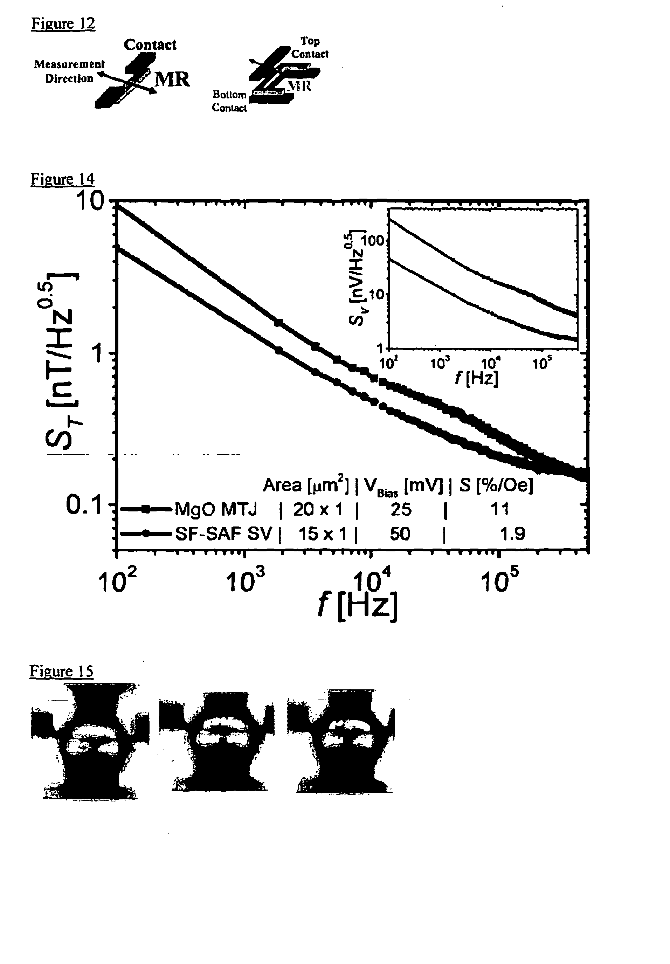

Figure 12 is a schematic of the GMR (LEFT) and TMR (RIGHT) sensors used.

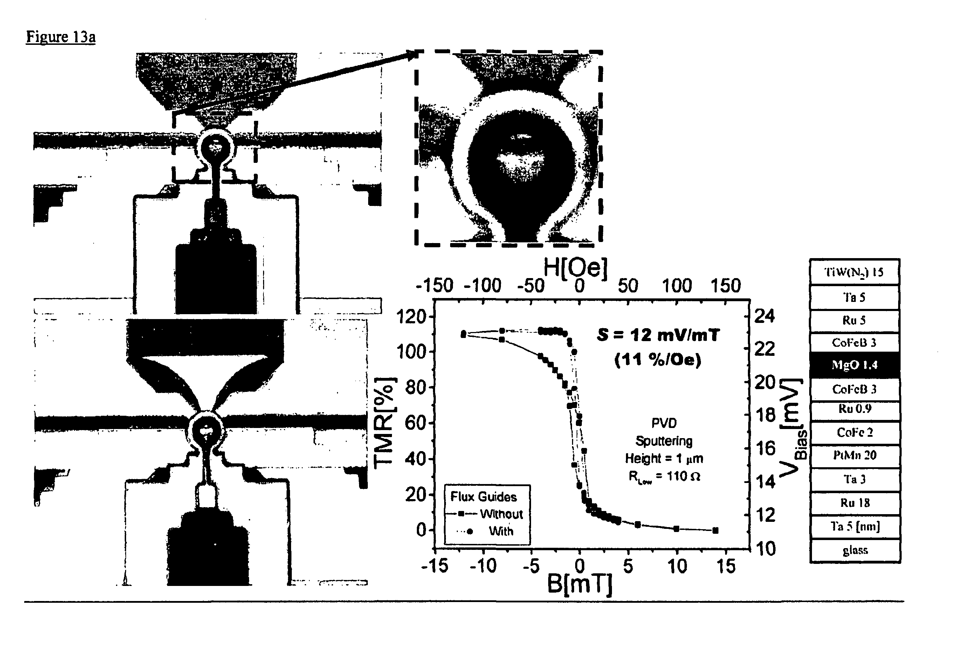

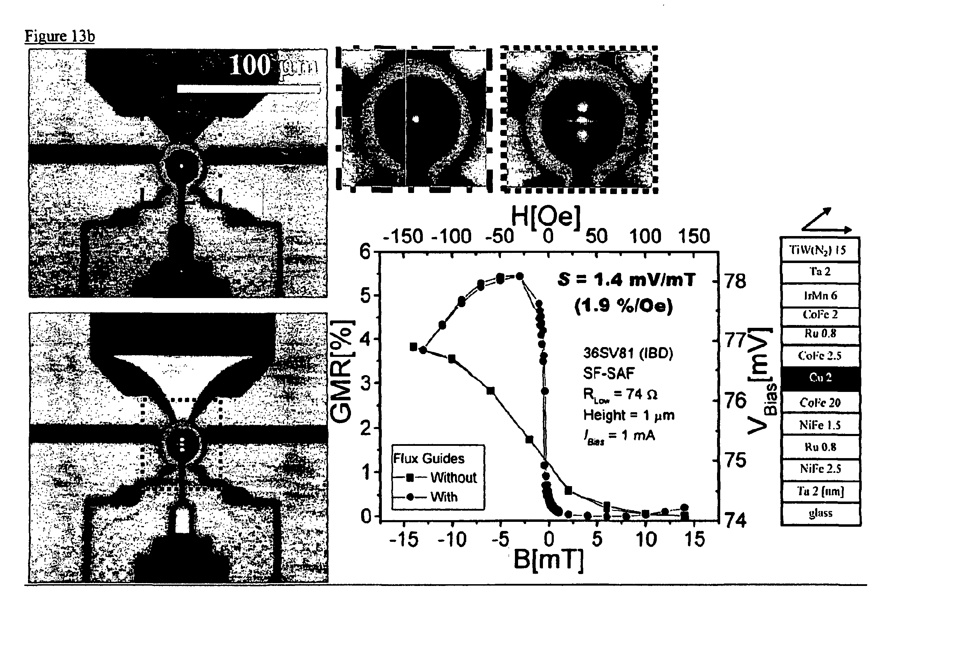

Figure 13 is an optical picture of magnetic tweezers integration in MR sensors, spin valves with and without flux guides (Figure 13a) and magnetic tunnel junctions (Figure 13b) in a 4 point contacts geometry.

Figure 14 shows field detection by the sensors used.

Figure 15 shows bead movement over the sensor due to applied current in the parallel current line.

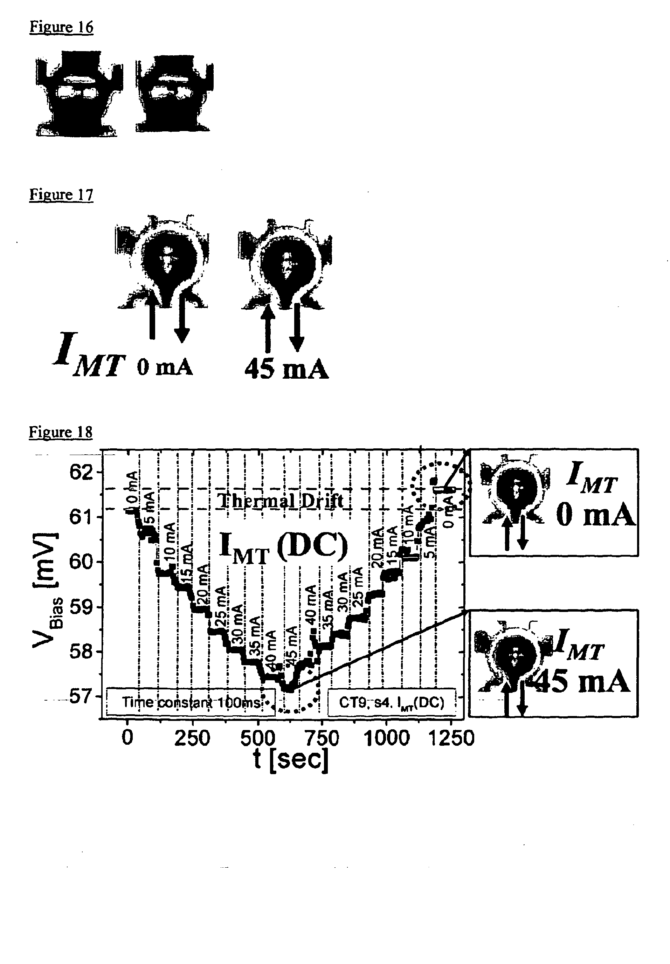

Figure 16 shows surface attachment of DNA. External Magnet applied to the chip surface in order to verify the DNA immobilization capability. The bead moves around the gold pad in a restricted area defined by the DNA length.

Figure 17 shows bead levitation promoted by the magnetic tweezers.

Figure 18 shows the readout (LEFT) associated to the tweezers operation (RIGHT).

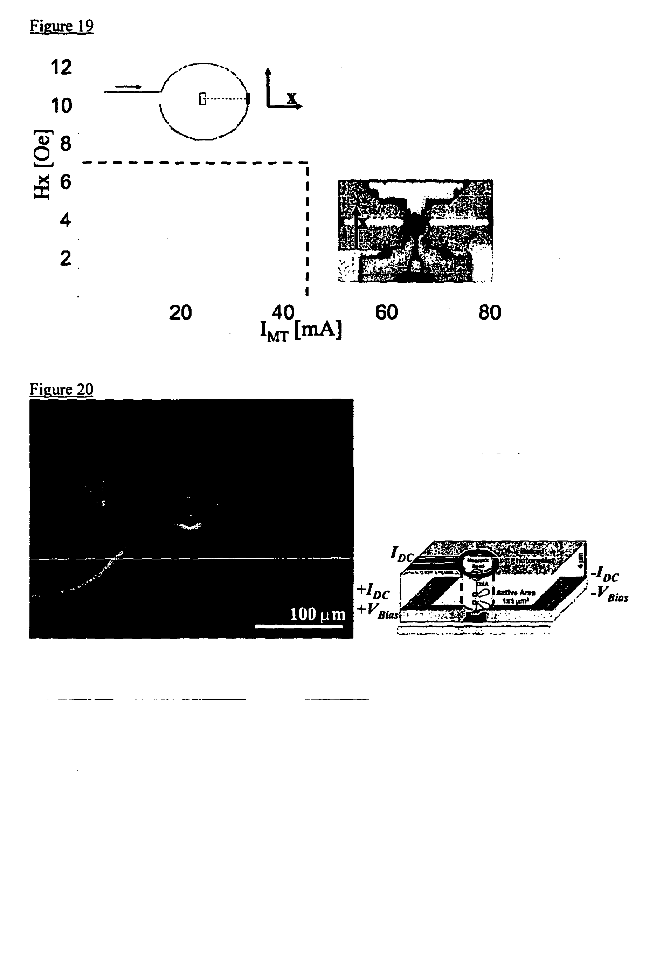

Figure 19 shows the transverse field created by the tweezers with ring shape geometry at the sensor level.

Figure 20 shows tweezers with a symmetrical geometry.

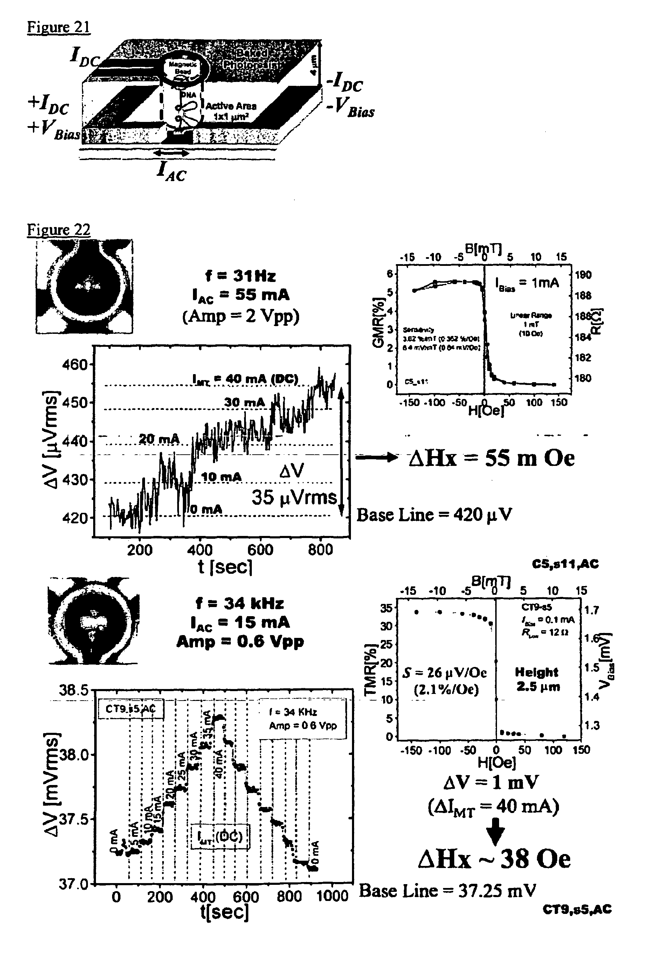

Figure 21 shows a schematic of AC current measured.

Figure 22 shows a readout using different bead excitation frequencies.

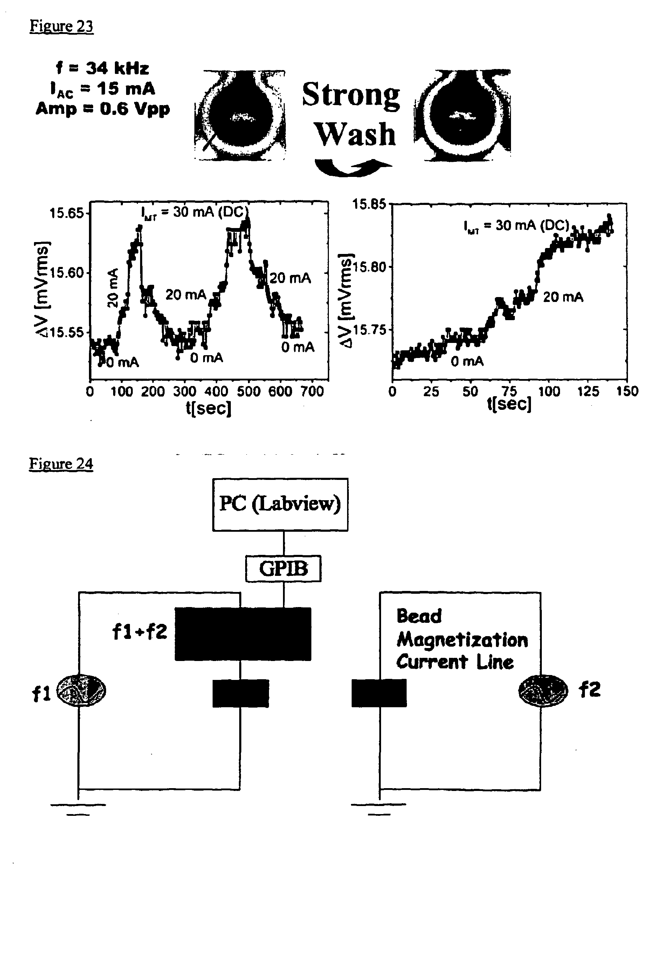

Figure 23 shows a readout with and without bead.

Figure 24 is a schematic of measurement made with both bead and current modulation.

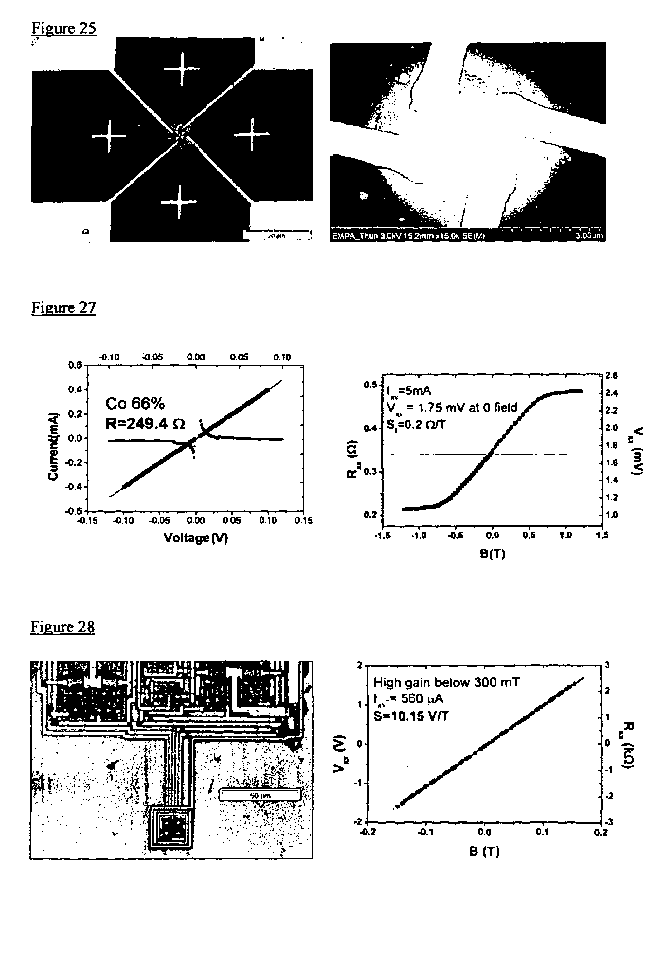

Figure 25 shows images of Hall Effect sensors. LEFT: Optical image of Hall sensor with Au electrodes on $Si0_2$/Si substrate: contact pads in the corners, Hall cross in the centre with e-beam made conductive wires in between. RIGHT: Zoom into Hall cross made of Co-C nano-composite deposited by maskless FEB. Active area below 1 x 1 $\mu m^2$. The width of current line is larger than the two voltage lines.

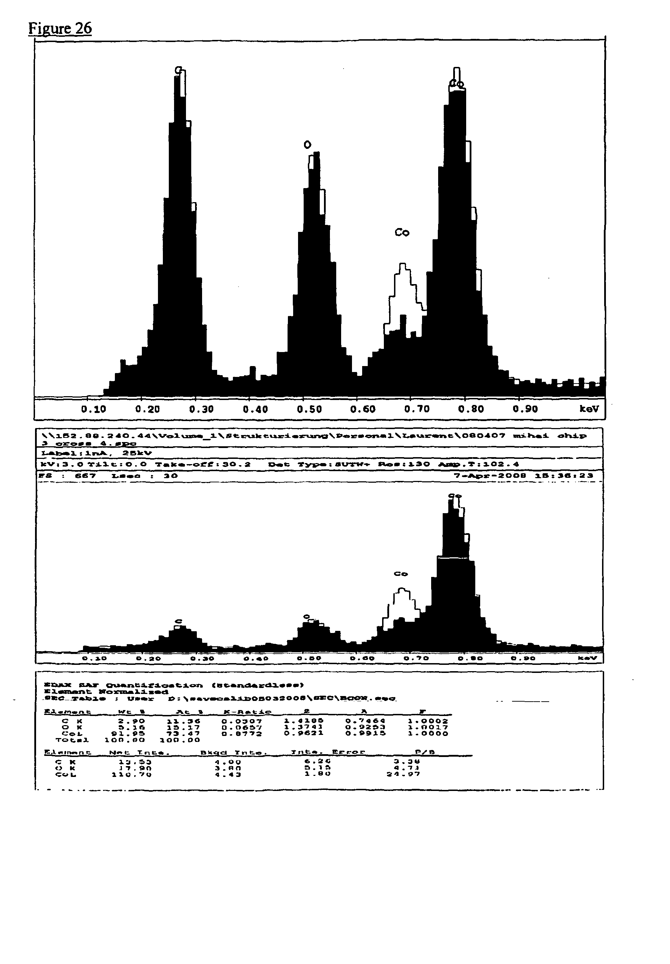

Figure 26 shows EDX spectra. TOP: EDX spectrum of Co-containing deposit from hexane-stabilized precursor. Deposit composition: 37 at.% cobalt (Co), 28.5 at.% oxygen (O), and 34.5 at.% carbon (C). BOTTOM: EDX spectrum of Co-containing deposit with improved deposition parameters. Deposit composition: 73.5 at.% cobalt (Co), 1 5 at.% oxygen (0), and 1 1 .5 at.% carbon (C).

Figure 27 shows measured Hall resistance (left) and Hall potential versus magnetic field (right).The sensitivity is 0.2 V/AT.

Figure 28 is an image of Hall sensor and measured resistance. LEFT: Zoom into modified region. Active area is approx. $2x2\mu m^2$. RIGHT: Measured Hall resistance and Hall potential versus magnetic field. Sensitivity is 10 V/T.

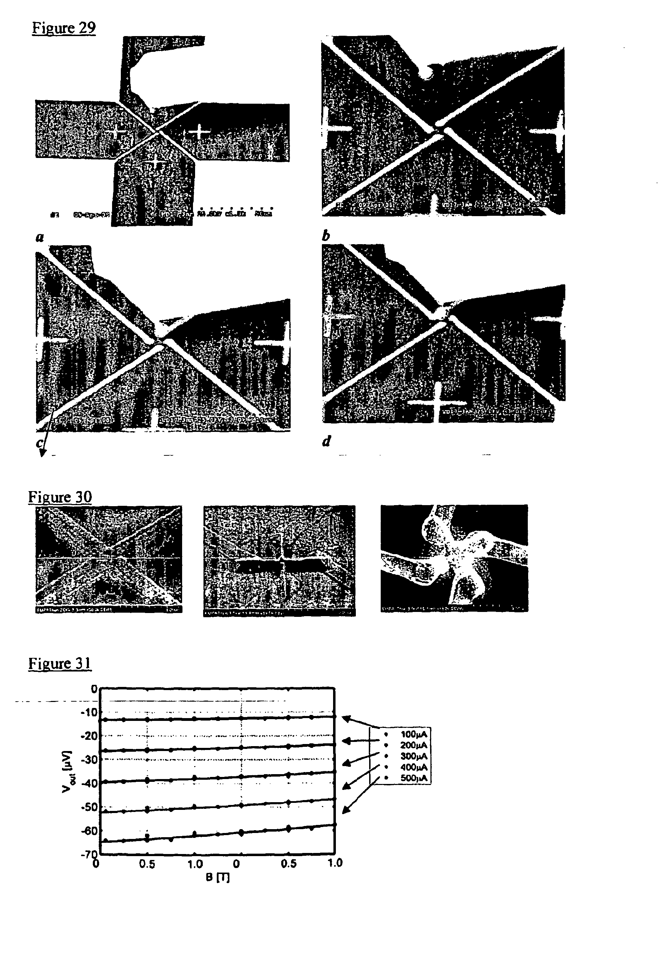

Figure 29 shows scanning electron microscope images of Hall sensors. SEM images of nanomanipulation setup for paramagnetic bead distance measurements, a) Overview of cantilever with paramagnetic bead (Dynabead: diameter 2.8 $\mu m$) above Hall sensor and gold electrodes, b) Close view of magnetic bead and Hall cross. Distance 11 $\mu m$ c) Bead approach: 1 .4 $\mu m$ close to Hall cross, d) Bead on Hall cross.

Figure 30 shows improvements to Hall sensors. LEFT: First sensors deposited with an average beam speed 0.5$\mu m$/3. The deposition dose is 5C/cm$^2$. MIDDLE: By increasing the beam speed while keeping the dose constant, the deposits become more homogeneous. The triangular cross section of the deposit is due to single scan deposition. RIGHT: By repeatedly scanning the beam over the deposition area with very high beam speed, the cross section of the deposit becomes rectangular.

There is no carbon-rich interface between current and voltage lines.

Figure 3 1 shows sensor response at different currents. Measured sensor response of first generation of sensors at different sensing currents. The deposited structures have a sensitivity of 13mV/AT and good linearity over the sub- Tesla range.

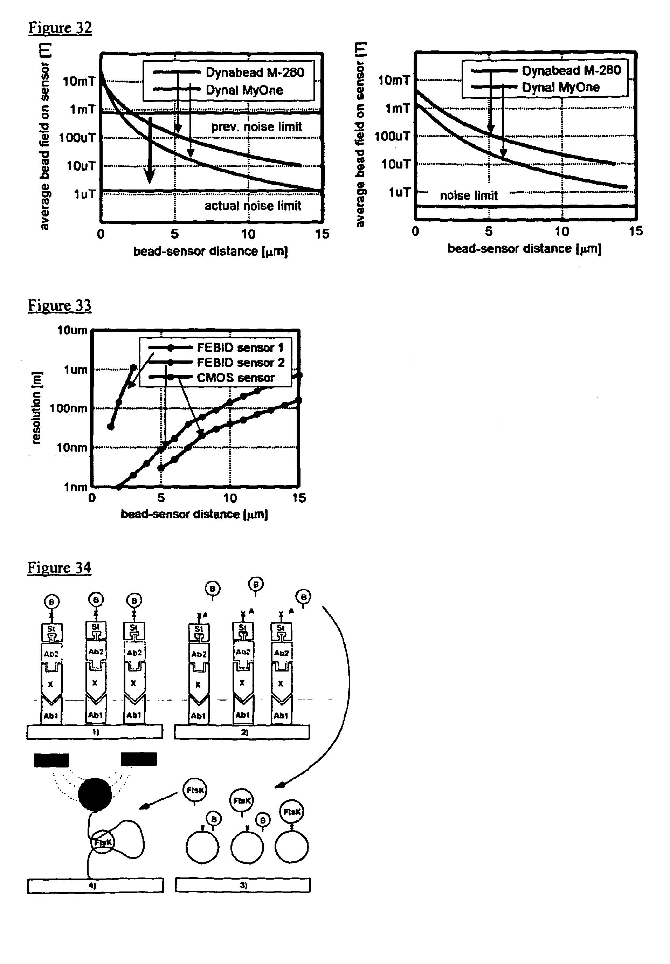

Figure 32 shows average bead magnetic fields on sensor surface. Average bead magnetic fields on sensor surface as function of bead-sensor distance. Beads: Dynabead M-280, saturated magnetic moment 1.7 I 0$^{-13}$ Am$^2$ and diameter 2.8 $\mu$m, and Dynal MyOne, saturated magnetic moment 2.3x 10$^{-14}$ Am$^2$ and diameter 1$\mu$m. Noise limits are indicated, a) FEBID sensors (active area 500x500 nm$^2$). The detection limit was set to the sensor's thermal noise: 770 $\mu$T/sqrt(Hz) (first generation) and 1.3 $\mu$T/sqrt(HZ) (second generation), b) CMOS integrated sensor (active area of 2.4x2.4 $\mu$m$^2$). The detection limit indicated is the measured sensor noise: 0.3$\mu$T/sqrt(Hz).

Figure 33 shows spatial resolution of sensors. Space resolution of first generation FEBID sensors (FEBID sensor 1), second generation FEBID sensors (FEBID sensor 2) and CMOS sensor. The data points are obtained by numerically solving the minimal detectable field as defined in Figure 32 for a corresponding variation in sensor-bead distance, assuming a measurement bandwidth of 1 Hz. The CMOS sensor is limited to bead-sensor distances above 5 $\mu$m due to its passivation layer preventing closer approach. At close distance, the thermal agitation of the bead (not shown) becomes the limiting resolution factor.

Figure 34 shows a cartoon describing a detection system:

Stage 1) Recognition of an antigen X in an ELISA sandwich assay with a protease B coupled to a streptavidin.
Stage 2) Release of the protease B by proteolysis of its bond (with protease A).
Stage 3) Release by protease B of a molecular motor (FtsK) or HsdR from EcoR124I [1].
Stage 4) Detection of the motor activity of FtsK through the induced motion of a microbead in a magnetic tweezer setup.

**Examples**

Example 1 - Amplification System

Overview

The amplification system has been described as a biological molecular amplifier and is viewed as an add-on kit for existing ELISA sandwich technology [2]. The biological molecular amplifier is shown in Figure 1. The biological molecular amplifier produces a light emitting output which can be measured in the same way as the output from a normal ELISA sandwich assay. Therefore, the device could be commercialised as an add-on kit to existing ELISA kits.

Stage 1 of the proposed device is a normal ELISA sandwich assay in which the antigen to be detected (X) is bound, within the "reaction chamber", by a surface attached antibody (Ab1) and then sandwiched between antibodies Ab1 and Ab2 following addition of Ab2 to the system. Antibody Ab2 is normally biotinylated and linked through a biotin-streptavidin system to a signalling system such as horseradish peroxidase, which will output light by cleavage of a chemiluminescent substrate. The inventors have adapted the biotin-streptavidin system for Ab2 such that a protease (Prescission™ Protease) is now linked to Ab2 through the biotin-streptavidin linkage. This linkage also includes a cleavage site for either thrombin or Factor Xa proteases (which are represented in Figure 1 as protease A) and, as can be seen from Stage 2, addition of either thrombin or Factor X to the ELISA sandwich results in release of the Prescission™ protease from Ab2 (providing that antigen X is present and bound in the sandwich). Each Ab2 molecule can bind up to three Prescission™ protease molecules through biotin-streptavidin linkages and the released protease then enters Stage 3 where it cleaves and releases an immobilised signalling enzyme such as Lux, GFP or EGFP. The inventors have determined that Prescission$^{1M}$ protease has a turnover of about 30, which means the amplification step of the process is around one hundred fold. In addition, the separate step, used for amplification, provides a reduction in background signalling due to non-specific binding of Ab2.

The inventors have also proposed that the antibodies used in this device could be readily replaced with aptamers [3, 4] (ssDNA or RNA molecules that can be "evolved" in vitro to bind to specific ligands). Attachment of the Prescission™ protease to the aptamer would be through a biotin attached to the DNA and this could be released by incorporation of suitable DNA sequence that would create a hairpin that produces a suitable restriction enzyme cleavage site, or as above using a protease site between the biotin-streptavidin link and the Prescission™ protease.

This system is a novel adaptation of the ELISA principle, which includes inventive steps used for amplification of the signal. See below for the detailed scientific description.

Detailed Scientific Description

The inventors used PreScission™ Protease, for protease B in Figure 1, based on the observations of its high specificity. What is required, therefore, is a biotinylated PreScission™ Protease with a cleavable linkage between the protease and the biotin. The inventors obtained a recombinant PreScission™ recombinant plasmid, which enabled cloning of the PreScission™ Protease next to either a thrombin site, or a Factor Xa cleavage site. A PCR product from this DNA has been produced and DNA cloning of this is now completed.

Since the ELISA sandwich system is produced using antibodies (= proteins), the inventors first focused on the problem of how to avoid the antibodies being cleaved by any protease used in Stage 1. Therefore, sequences of fragments of Immunoglobulin heavy chain variable regions and Immunoglobulin light chains (Mouse and Rabbit), available in the UniProt Knowledgebase, were analysed for their sensitivity to Enterokinase, Factor Xa, Thrombin and PreScission™ Protease, using the Peptide Cutter tool. A random screen of 40 sequences revealed cleavage sites only for Thrombin (twice) and Factor Xa (once), This is quite promising, considering the great immunoglobulin variability and it reflects the strong specificity of the chosen proteases.

The inventors have developed a fluorescence assay, based on cleavage of a peptide, which will provide a calibrated system for measuring proteolysis to allow exact determination of turnover. However, in the meantime, the inventors now have available the GST-(PreScission-site)-HsdR fusion substrate for cleavage by Prescission™ protease and have demonstrated specific release of HsdR from this fusion protein, which in turn has allowed the inventors to make a determination of turnover and the degree of possible amplification.

The inventors analysed the SDS-PAGE gel (Figure 2) to quantify the release of HsdR from the GST-(p- )-HsdR fusion protein. The cleavage was most easily monitored by measuring the % of GST present on the gel (Figure 3). Complete digestion was observed after 90 min when incubated with 1 Unit of Prescission™ protease. This equates to a total of 32 HsdR subunits released per molecule of protease.

Vectors pGL4.10 and pGL4.73 were obtained from Promega. pGL4.10 encodes a synthetic firefly luciferase gene and pGL4.73 encodes a synthetic Renilla luciferase gene. The inventors have also obtained a vector carrying a variant of the "Green Fluorescence Protein" reporter gene - Enhanced GFP - which the inventors can introduce a cleavage site into. Primers have been designed and PCR amplification experiments are completed, the inventors have ligated each gene into each of the three available pGEX vectors, in order to produce GST-(cleavage site)- luciferase EGFP with each of the protease cleavage sites. These clones can then be used as templates for production of cassettes for downstream cloning experiments, for incorporation of different tag systems etc. Analysis of the recombinant DNA suggests that the inventors have available a GST-(Pres.Prot site)-EGFP.

The thrombin binding aptamer [5], as one of the first aptamers to be studied, is well defined in its adapted secondary structure and the interactions of aptamer and antigen are almost fully characterised.

In order to characterise any changes to the interaction of the thrombin binding aptamer to thrombin when attached to a surface, Surface Plasmon Resonance (SPR) studies were performed. These studies aim to corroborate previous data [6] and determine the ability to detect and quantify thrombin within a solution as well as confirm aptamer-ligand binding is NOT affected by surface attachment. Three aptamers were designed using the consensus sequence drawn from Bock et al. the affinity of the target to each individual ligand being relatively strong, medium, and weak respectively.

Aptamer sequences used:

Thrombin Aptamer v1 : GGTAGGTGTGGTTGGTGCAAC (SEQ ID NO.1)
Thrombin Aptamer v3 : GGTAGGTTTGGTTGGTGCAACGC (SEQ ID NO.2)
Thrombin Aptamer R70EssP5: GGTAGGTAGGTATGGTGCAACGC (SEQ ID NO.3)

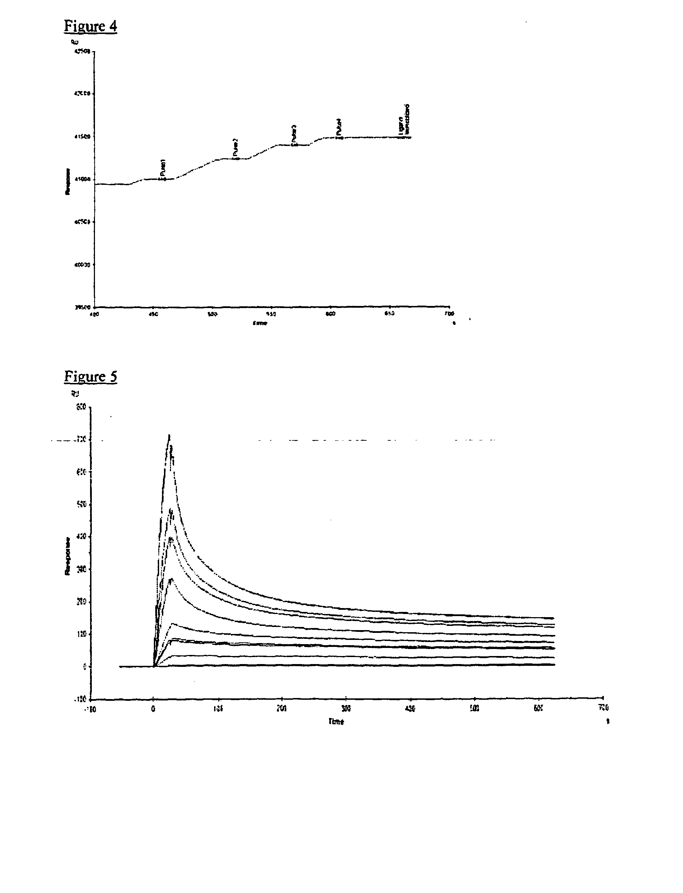

Figure 4 depicts the surface attachment of aptamer v1 via a biotin-streptavidin interaction. The streptavidin coated SPR sensor chip and the 5' biotinylated aptamer v1 were incubated together with a low flow rate across the chip until the response units reach the required level. Injections of the aptamer solution fall at time points 410 s, 520 s, 570 s, 610 s with the desired level of response achieved after 675 s.

In Figure 5 increasing concentration of thrombin are observed to rapidly bind the surface-attached aptamer, followed by a slow dissociation of the ligand from the aptamer indicative of the expected tight binding.

Example 2 - Sensing Apparatus

Overview

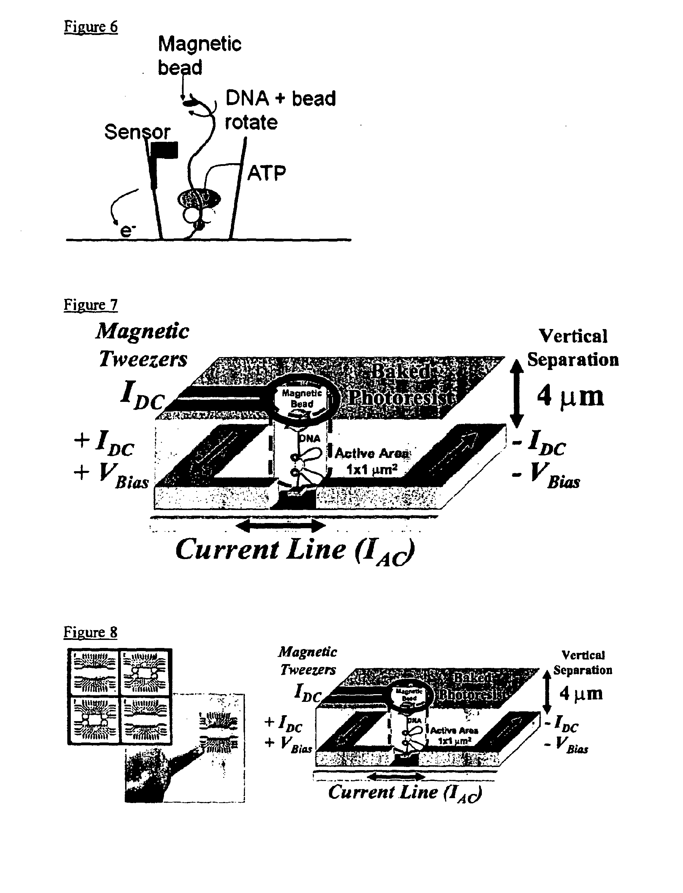

The sensing apparatus has been described as a molecular switch device that links the biological and silicon worlds. The idea is that a biological molecular motor would 'pull' a magnetic bead attached to DNA and, therefore, output an electronic signal from what is effectively a molecular dynamo (Figure 6). In this device, a single strand of DNA is attached to the surface of a flow cell in a manner analogous to that used in magnetic tweezer systems, a bead is attached to the other end of the DNA and this bead is pulled past a sensor, which outputs an electronic signal from the biological input (ATP), which is the fuel for the motor.

The key to the development of this device has revolved around the application of two different sensing technologies for detecting the vertically moving magnetic mead.

The first sensor to be described was a Hall Effect sensor which was briefly trialled and provided sufficient Proof of Concept to justify further work. The inventors also introduced the concept of using magnetoresistive devices as the sensor. The magnetoresistive device has proven to be a very capable device and a possible design for the device is shown in Figure 7.

The key development within this device is the use of a simple coil to hold-up and manipulate the magnetic bead, which will allow stretching of the DNA attached to the base of the device in a manner similar to that described for a magnetic tweezer setup. Pre-prototype versions of this device have been produced and the scientific detail is presented below.

Detailed Scientific Description

GMR (giant magnetoresistive) and TMR (tunnel magnetoresistive) effect based sensors, in particular MgO based magnetic tunnel junctions (with TMR ratios up to 200% and RxA tuneable between a few ohms and several hundreds of kilo ohm per micron square), with synthetic free and pinned layers and also incorporated with magnetic flux guides with gain up to 40 times without increasing the noise level of the sensor, have been used to detect the presence of magnetic beads and also shown to be capable of measuring the vertical displacement.

Incorporating into the device a current line with a ring shape at a certain separation above the sensor a magnetic gradient field allows bead levitation, this solution results in a full in-chip platform. The main draw-back of this geometry is the fringe field associated to the vertical DC magnetic field created at the sensor level by the ring, which implies the use of a punctual or symmetric sensor measuring only the in-plane component of the bead fringe field. In order to avoid the magnetic noise background, the bead is magnetized at a certain frequency, for that the inventors propose an in-chip current line parallel to the sensor is used, frequencies of hundreds of kilohertz are allowed, depending on the dimension of the current line and the characteristic electrical length associated to the frequency used, typically hundreds of kilohertz. To avoid the cross-talk between the bead excitation circuit and the sense element, the sensor is being used in AC mode, and the product of the modulation is measured using a Lock-In amplifier. Due to the heat dissipation (power up to 50 mW) associated to the magnetic tweezer, Wheatstone bridge configuration is being used in order to avoid thermal drift in the sensor readout. The electrical system must be properly passivated in order to be used in physiologic conditions. Therefore, the passivation is being optimized incorporating an aluminium nitride layer to resist to sodium ions, which penetrate into oxide layers. In addition, PDMS chambers are being integrated in order to allow the use of molecular motors and to improve the stability of the measure.

As an alternative to the PicoTwist system used to stretch the DNA, with a magnetic gradient of 1 Tesla / mm which creates a transverse component at the substrate level of ten milliTesla (one hundred oersters) saturating an in-plane sensor like a MR sensor, the magnets could be used in a vertical configuration. In this case, a ring shape sensor could be used to measure the vertical component of the magnetized bead fringe field; however, the measurement would be done in DC mode in order not to interfere with the bead vertical position. Thus, the inventors propose to integrate a current line in a ring shape above the sensor, Figure 8, which creates a magnetic field at the bead level resulting in a magnetic force strong enough to levitate the magnetic bead stretching the DNA strand. A transverse magnetization component could then be created on the bead by an additional current line, parallel to the sensor, allowing AC modulation.

To integrate a magnetoresistive sensor in this system in order to measure the end-to-end distance of a single long DNA molecule, bound at one end to a magnetic bead and at the other to the surface, first together with the optical system complementing the optical information, then in a second approach using only the MR sensor in a full miniaturize platform, magnetic tweezers based on an aluminium current ring and magnetic cladding (NiFe 0.1 $\mu$m) with 10 $\mu$m diameter were integrated with magnetoresistive sensors, GMR and TMR, and magnetic flux guides. The two structures were coupled through a 3, 4 and 5 $\mu$m photoresistive multilayer mesa and a hole of the same internal diameter of the tweezers which was defined by photolithography, Figure 8. The photoresistive was cured by infrared rapid thermal anneal (without magnetic field applied) in a high vacuum chamber, where 225°C are achieved in 50 seconds. This procedure allows the different crystalline anisotropic axes of magnetic structures underneath.

The predicted magnetic force applied on a magnetic bead is 1 pN, under a field of 5 mT, which allows bead levitation. The characterization of the tweezers and the operation with super paramagnetic beads linked to long DNA, 10 kbp, functionalized in gold pads of 1 x 1 $\mu$m$^2$ was being tested using sensors with sensitivities of 2%/Oe, GMR, and 11 %/Oe, TMR. Using glass substrates, contacts with 1 $\mu$m height and sensors with heights down to 70 nm, real-time optical and electrical observations were possible. The platform is scalable and can be adapted according to application-specific requirements.

The magnetic beads used have super paramagnetic properties which imply that in the presence of an external magnetic field, a magnetic moment is induced in the beads, which follows almost instantly the applied field. When the field is removed, the labels quickly lose their magnetization. Furthermore, super paramagnetic beads can be magnetized to large magnetic moments, facilitating label transport by magnetic actuation techniques. As a consequence of the fast super paramagnetic relaxation, the inventors need to have a magnetizing field to detect the bead. In a first approach, micrometer-sized labels (3 μm) are being used allowing the visual verification of label position using visible light microscopy.

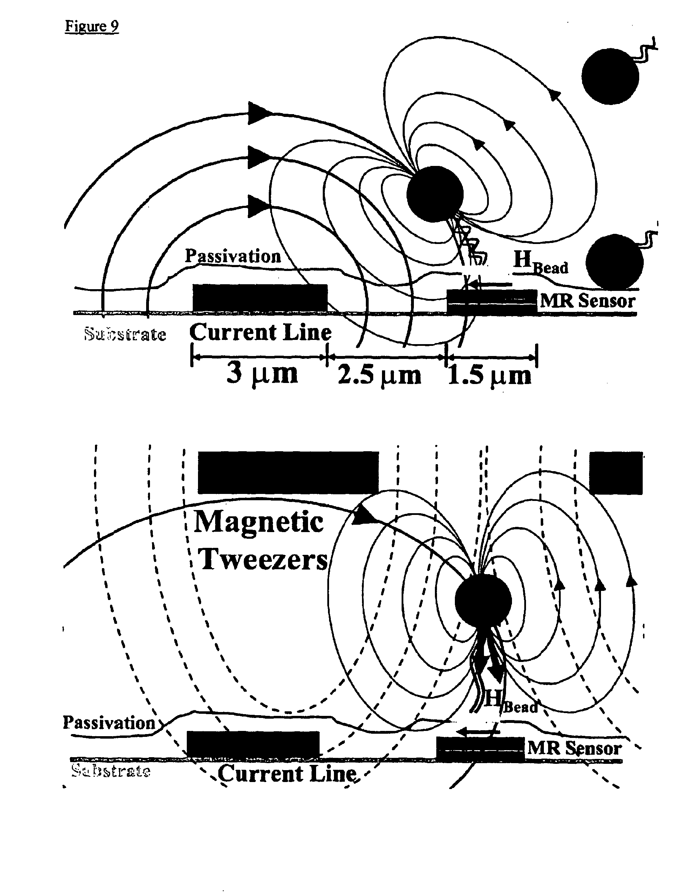

In order to detect the fringe field, created by the magnetic field, the bead is magnetized in-plane resulting in a transversal component created by the bead at the sensor level. For that purpose, parallel current lines are being used, Figure 9. Integrated in the substrate of the sensor chip, the current lines are used to generate a well-defined magnetic field on the sensor surface, thus removing the need for mechanical alignment with external apparatus (external magnets or coils). Moreover, the dimensions of the field-generating circuitry and the detection circuitry can be made small. This allows the modulation in frequency of the magnetization of the bead up to hundreds of kilo hertz, optimizing the signal-to-noise ratio. In this case, the inventors can just pick up the signal associated with the frequency of operation, avoiding the background noise of the setup. The current lines present in the platform, for magnetization of the beads in the magnetic tweezers setup (with a ring shape centre on top of the sensor) are used to move and position beads on top of the sensors.

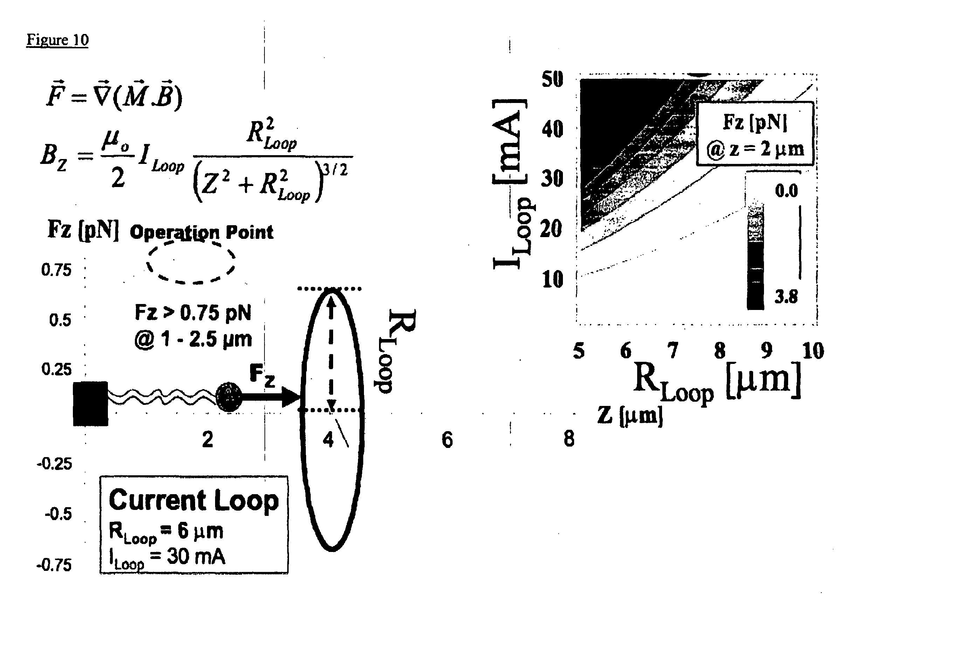

The tweezers were designed in order to optimize the force applied in the magnetic bead. The main request for the magnetic tweezers is the vertical magnetic force at the bead level. In addition, this force must be the most CONSTANT as possible in order not to interfere with the molecular motor operation. This is established by the geometry: separation, diameter and ring inner radius. The intensity also depends on the geometry used, but can be tuned with the current used. Using DNA of 1 .5 μm length and Beads of 3 μm, to create a magnetic force of 1 pN at a vertical distance of about 2 μm, a current line in a ring shape with 12 μm of diameter at a separation of 4 μm were used, Figure 10. With current up to 50 mA the force required is achieved.

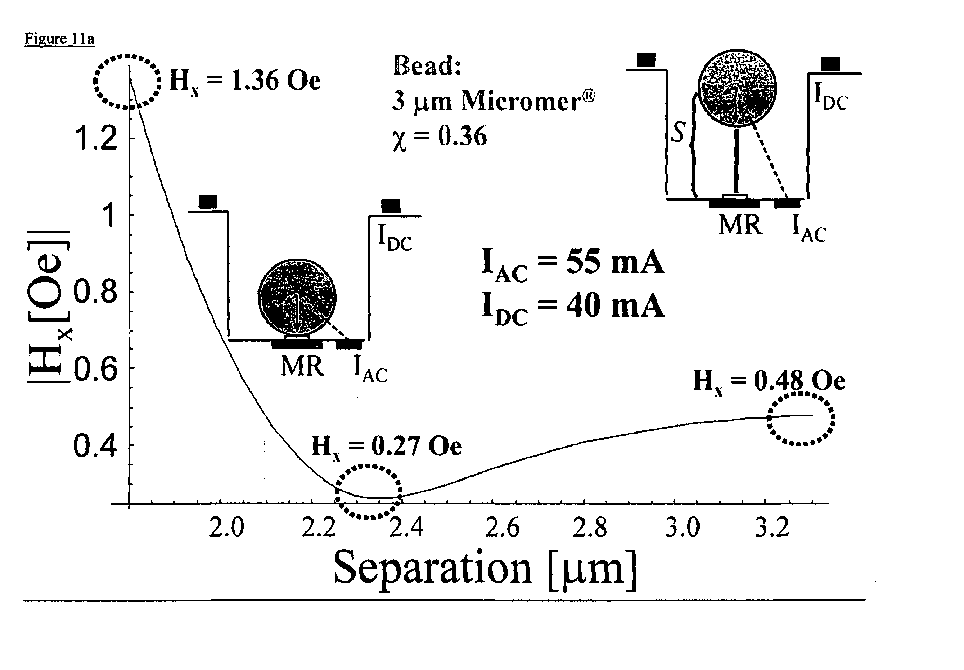

Regarding the fringe field created by the bead. Having a tweezers operation with $I_{DC}$ = 40 mA, and $I_{AC}$ = 55 mA in the magnetization line, at distance of 2-3 μm above the sensor, one bead with 3 μm of diameter creates a fringe field at the sensor level of about 50 microTesla, therefore, a variation in the vertical position of 1 nm implies a field change of 40 nanoTesla, Figure 11.

In order to detect this kind of field MR sensors were implemented. The MR sensors are sensitive mostly to the field- component along the transverse direction in the free magnetic layer, Figure 12.

Therefore, a sensor strip flanked by field-generating conductor strips is most favourable. In this arrangement, the excitation field of the wires is perpendicular to the sensor plane along its complete length, while the stray field of a magnetized bead (label) produces a field-component in the plane of the sensor strip. In this way, large magnetizing fields can be applied to the beads without saturating the sensor, while the very small stray fields of the magnetized beads can still be detected.

MR sensors have good sensitivity to small magnetic fields, such that high signal-to-noise ratios (SNR) can be realized with submicron-sized beads. Moreover, stacks of appropriate materials can be deposited on glass substrates with nanometre scale surface dimensions.

The challenge of the tweezers integration is the fact that it needs to be located above the sensor within a range of a few microns, and after that the inventors still need to continue the assembly processes in order to proper insulate the system and define an element (typically gold) for the immobilization of the DNA.

Glass substrates are being used in the platform in order to be transparent for combined optical measurement of bead movement. The sensors were deposited by Ion beam deposition and sputtering. The sensor element is defined by electron lithography or photo lithography and by ion beam milling. The metal contacts to the sensor are defined by lift-off. In order to create a separation between the sensor and tweezers, a baked photoresistive mesa were used. The sample is coated with 3-5 layers of photoresistive material 1.5 μm each, and exposed with a direct write laser system. After that the photoresistive material is cured in order to support the following process.

For that propose, an infra red system was used, where a rapid thermal anneal in an ultra high vacuum chamber is performed. Almost two hundred degrees in forty seconds are achieved. After that, the tweezers are defined by lift-off. The system is passivated in order to be compatible with physiological conditions. Nitrides are commonly used as they provide a good barrier to sodium ions in physiological environment, which penetrate into oxide layers and destroy their insulating properties. However, under some circumstances a nitride layer alone is not suitable. For instance, in physiological saline solution, under an applied electric field such as may result from active components on a chip, nitride rapidly degrades. So a multiple layers of oxide and nitride are used. In order to allow the integration with a PDMS chamber an additional layer of $SiO_2$ is required, with a minimum thickness of 0.2 μm. In order not to increase the separation between sensor and bead, a frame of $SiO_2$ is defined only in an area at the boundary of the chamber walls.

In order to immobilize DNA precisely on top of the sensor element and in centre of the tweezers, a gold pad is defined by lift-off. Figure 13 is an optical picture of the final device processed.

The sensors used have 4-contact geometry in the case of magnetic tunnel junctions and 2-contact geometry in spin- valves. In a spin-valve the inventors can set the spatial resolution by the height of the sensor and by the distance between contacts, which will define the minimum bead size detection capability. However, in a MTJ, since the current must flow perpendicular to the plain, the spatial resolution must be set by the contact area to the sensor with an additional oxide layer, Figure 12. The use of narrow geometries will also allow the integration with an inverted microscope system.

MR sensors are affected by low frequency noise, Figure 14. The magnetic 1/f noise is the dominant noise component. With increasing frequency the magnetic noise component in the MR voltage decreases. For this reason, it is preferred to generate a magnetic field for bead excitation at a frequency higher than the 1 /f cut-off frequency, so that the inventors can expect the smallest possible noise contribution from the sensor.

In order to improve the field sensitivity, magnetic flux concentrator can be integrated without increasing the intrinsic noise level of the sensor.

Upon applying a DC current to the current line parallel to the sensor, the one used to magnetize the beads, and switching the current ON and OFF the beads are attracted and placed above the sensor, Figure 15.

Long DNA, with 5 kbp connected to biotins and thiol groups were used together with micrometer beads with 3 microns of diameter coated with streptavidin.

Modified DNA stands with thiol groups are label with magnetic beads throw the interaction biotin-streptavidin and then immobilized in the gold surface. Which can be verified using a permanent magnet underneath the chip, and rotating it, the inventors can observe all beads rotating, unless the ones binding to the gold pad, Figure 16, which moves only in a restrict area defined by the DNA length.

With a bead immobilized in a gold pad of $1 \times 1$ $\mu m^2$ above a MR sensor, pulsed current with intensity of 45 mA were applied to the magnetic tweezers, the bead is clearly levitated by the force gradient created by the tweezers, Figure 17.

During the bead movement, associated to the current applied in the magnetic tweezers, the readout in the MR sensors underneath, Figure 18, indicate a dc coupling between the magnetic tweezers and the MR sensors.

For the example indicated a sensor with a field sensitivity of 320 uV/Oe were used, therefore, the voltage change of 4 mV associated to the difference between the upper and lower bead position, promoted by a variation of 45 mA in the tweezers above the sensor, has a equivalent transverse field of 12.5 Oe. The field measure arises directly from the geometry of the tweezers used, Figure 19.

This contribution could be compensated using a full symmetrically geometry, Figure 20.

The thermal drift observed in the baseline of the readout is due to the power dissipated in the magnetic tweezers, up to tens of mW. In the present generation of platforms Wheatstone bridges sensors are being implemented allowing differential measurements and more resistance to thermal drifts.

Turning ON the parallel current lines in order to magnetize the bead with a certain frequency, Figure 21, and picking up the signal with a Lock-In amplifier in the readout, the inventors observed that high-frequency excitation currents unintentionally induce a voltage and current in the sensor. This results in a parasitic crosstalk voltage at the excitation frequency that is orders of magnitude larger than the signal to be expected from magnetized beads. This scales directly with the excitation frequency, Figure 22.

This coupling almost saturates the sensor and vanish the sensitivity. However, the bead presence is detectable; this is confirmed repeating the measurement with and without magnetic bead, Figure 23. Using a sensor with a sensitivity of 265 $\mu v$/ Oe, a excitation frequency of 34 kHz with an amplitude of 15 mArms the voltage change associated to a tweezers current of 30 mA in the case of the bead is about 100 $\mu V$ and without bead is 70 $\mu V$. This difference of 30 $\mu V$ has a equivalent transverse field of 110 mOe which is in agreement with the predict value for this geometry and magnetization values.

Apparently, to reduce the dynamic range of the sensor voltage, the magnetic signal should be separated from this electromagnetic crosstalk voltage. To that end, the sense current will be modulated as well, Figure 24.

The inventors have focused on developing a sub-micron sized Hall sensor suitable for precise magnetic bead tracking as a tool to monitor DNA-elongation. The inventors investigated the use of focused electron-beam induced deposition (FEBID) of Cobalt as sensor material, as these deposits have been shown to exhibit a large extraordinary Hall Effect. The actual sensors exhibit a Cobalt content of 70%, at a resistance of 100 Ohm and sensitivity to magnetic field of 0.2V/AT. This allows tracking of super paramagnetic microbeads over several micrometers above the sensor.

Alternatively, the performances of CMOS integrated Hall sensors were investigated. The measured sensitivity of 10 V/T shows that this type of sensor would also be capable of magnetic bead detection.

Using an electron microscope based nano-manipulation tools, the inventors attached single paramagnetic / magnetic beads to AFM tips which can be used to mimic the action of the restriction enzyme on the DNA (which will pull the paramagnetic bead to the sensor by curling up the DNA).

The inventors have also addressed the resistivity problem they had with the first generation of FEBID fabricated sensors. It is important to reduce the resistivity below 10 kOhms in order to increase the maximum DC current, reduce the risk of electrostatic discharges, and to decrease the noise during measurements.

Lithography and FEBID of magnetic sensors

The first step in improving the conductivity of the Focused Electron Beam induced deposited Co layers was to reduce their length, by reducing the gap between the conduction pads. The inventors have fabricated their own photo-lithography masks which they used in combination with e-beam lithography to get electrode gaps below 1 $\mu$m. In the same time the inventors have increased the dimensions of the contact pads in order to use either wire bonding or pogo pins to contact the samples, see Figure 25.

The reduced length of the wires was beneficial for reducing the resistance according to Ohm's law and also for increasing the Co content, since the amount of Co in the deposit depends intrinsically on the e-beam writing strategy. In order to investigate further this dependence the inventors have carried out energy dispersive X-ray (EDX) measurements both on old and new samples, with different lengths and writing strategies, see Figure 26. The inventors have also discovered an intrinsic problem of FEBID depositions done with precursors stabilized in hexane, which was a high contamination with carbon, both at the interfaces between the wires and on the conductive pads. Argon-stabilized precursors are less prone to this problem, and, combined with a writing strategy which consists in repeating the whole structure to get a certain dose rather than writing once each wire, resulted in deposits with high Co content (73%) and low resistance (below 100 ohms for 2 point measurement of the current line).

Resistivity and Hall measurements of FEBID fabricated magnetic sensors

The inventors have carried out both resistivity and Hall measurements on a set of samples with high Co ratio. Although the two point probe resistance measurements include the contact resistance, the inventors could measure typical wire resistances values around 100 ohms. Measured Hall sensitivities varied from 0.02 V/AT to 0.2 V/AT depending on the wire thickness. For an example see Figure 27.

The next step in the inventors approach was to correlate the Co content with the wire resistivity.

Hall measurements of CMOS integrated magnetic sensors

Alternatively, the inventors investigated the performances of CMOS integrated Hall sensors [7]. The measured sensitivity of 10 V/AT shows that this type of sensor would also be capable of magnetic bead detection, see Figure 28.

Magnetic bead detection setup

In order to test the sensitivity of the magnetic sensors the inventors want to detect the movement of a paramagnetic bead above the sensor. To do so the inventors attached a bead on an AFM tip which itself is fixed to a nanomanipulator with precise movement control. This setup can be used to mimic the action of the restriction enzyme on the DNA which will pull the paramagnetic bead to the sensor (by curling up the DNA).

The SEM images in Figure 29 show the approach of a bead towards a FEBID-deposited Hall cross. This test is also compatible when using an insulating layer on the top of the Hall crosses which will be necessary in order to put the Au layer necessary to attach the DNA above the sensor. Both the passivation and the deposition of the Au layer can be done using (in situ) FEB induced deposition.

Improvement of FEBID writing strategy

First series of deposited Cobalt containing sensors showed a resistance of 1.5 kOhms and a magnetic sensitivity of 13mV/AT. The resistance corresponds to a resistivity of $10^{-5}$ Ohm.m, i.e. three orders of magnitude higher than bulk Cobalt, pointing to a high content of carbon in the deposits. The first batch of sensors was deposited using point-by-point writing. By adapting the deposition writing strategy, more homogeneous sensors were achieved. As deposits are surrounded by a protective carbon-rich matrix, this helps decreasing resistivity of the sensors by removing low- cobalt-content interfaces inside the deposited structure, see Figure 30.

Improvement of magnetic sensitivity

Improvement of magnetic sensitivity from 13 mV/AT to 0.2 V/AT was achieved during the first project period. Hall voltage output of the first low-sensitivity sensor series is shown in Figure 31.

Theoretical calculations of the magnetic bead field detected by the sensor show the feasibility of detection of moving paramagnetic beads.

The inventors performed calculations of the bead field on the sensor based on the magnetic dipole model:

$$\vec{B} = \frac{\mu_0 m}{4\pi} \frac{3(\hat{m} \cdot \hat{r})\hat{r} - \hat{m}}{r^3},$$

with $\mu_0 = 4\pi * 10^{-7}$ Vs/Am: permeability of vacuum, m: magnetic moment of dipole [Am$^2$], r: vector from dipole centre to point in space, and '^' denoting unit vectors. As the Hall sensor is only sensitive to components of the magnetic field perpendicular to it, only the field in z direction is considered:

$$B_z(x, y, z) = \frac{\mu_0 m}{4\pi} \frac{2z^2 - x^2 - y^2}{\left(x^2 + y^2 + z^2\right)^{5/2}}$$

The average bead field on the sensor is then computed by:

$$\overline{B}(z) = \int\limits_{-w/2}^{w/2} \int\limits_{-w/2}^{w/2} B(x, y, z) dxdy \left/ \int\limits_{-w/2}^{w/2} \int\limits_{-w/2}^{w/2} dxdy \right.$$

where w is denoting the width of the active rectangular sensor area. Calculations were performed for the FEBID sensors (active area 500 nm x 500 nm) and the CMOS sensor (active area 2.4 $\mu$m x 2.4 $\mu$m) and are shown in Figure 32. A fully saturated, micron-sized bead could be sensed up to a distance of several microns. Figure 32 shows how the reduction in sensor resistance from 1.5 kOhm to 100 Ohm, coupled with an increase of sensitivity from 13 mOhmT to 200 mOhm/T, drastically enhances the resolution limit of the sensor.

The ultimate resolution of the magnetic sensor is limited by the intrinsic thermal noise of the sensor given by:

$$VN = \sqrt{4 . k . T . R.} \sqrt{\Delta f}$$

where $V_N$ is the thermal noise RMS [V], k is Boltzmann's constant (1.4x 10$^{23}$ J K), T is the temperature ( ), R is the resistance of the sensor (Ohm) and $\Delta f$ the measurement bandwidth (Hz). This intrinsic noise translates to the minimal detectable magnetic field through the sensor's sensitivity, using the following relation:

$$B_{min} = V_N / (S_0 . I_{meas}),$$

where $B_{min}$ is the minimal detectable field [T], $S_0$ is the sensor's sensitivity [V/AT] and $I_{meas}$ is the measurement current applied [A]. The minimal detectable magnetic field corresponds to the smallest variation in position of the bead which the sensor can detect. Thus, the space resolution of the sensor can be obtained by solving the following equation:

$$B_{mm} = \Delta \overline{B}(z, z_{min}) = \overline{B}(z + z_{min}) - \overline{B}(z),$$

where $z_{min}$ [m] is the minimal detectable change in the bead's position, considering a position z[m].

The actual noise level of the sensor will have to be assessed experimentally as it depends also on other noise sources, such as shot noise or flicker noise. These other noise sources were shown to increase the noise level by about one order of magnitude for FEBID sensors, or just a factor of two when using adequate low-frequency noise suppression techniques.

Summary and outlook

The inventors have shown the ability to deposit reproducibly Hall structures with over 70% Cobalt content, low resistance ( 100 Ohm) and good sensitivity (0.2V/AT), thus pushing the detection limit below the expected magnetic field of the super paramagnetic microbeads to be tracked.

Example 3 - Detection System

The detection system or biosensor comprises the amplification system and the sensing apparatus described above and a conventional ELISA sandwich assay as the "front-end" of the biosensor. A cartoon of the biosensor is shown in Figure 34.

The biosensor is based around a transducer signalling system that operates at the single molecule level and detects a released biological molecular motor through movement of a magnetic bead, attached to DNA, which is 'pulled' by the biological molecular motor (Stage 4 of Figure 34).

**References**

1. Firman, K. (1999). A polynucleotide motor. United Kingdom Patent, GB2351083. University of Portsmouth.

2. Kato, K., et al., Use of rabbit antibody IgG bound onto plain and aminoalkylsilyl glass surface for the enzyme-linked sandwich immunoassay. J Biochem (Tokyo), 1977. 82(1): p. 261 -6.

3. Huizenga, D.E. and J.W. Szostak, A DNA aptamer that binds adenosine and ATP. Biochemistry, 1995. 34(2): p. 656-65.

4. Lauhon, C.T. and J.W. Szostak, RNA aptamers that bind flavin and nicotinamide redox cofactors. J Am Chem Soc, 1995. 117(4): p. 1246-57.

5. Bock, L.C., et al., Selection of single-stranded DNA molecules that bind and inhibit human thrombin. Nature, 1992. 355(6360): p. 564-6.

6. Tang, Q., X. Su, and K.P. Loh, Surface plasmon resonance spectroscopy study of interfacial binding of thrombin to antithrombin DNA aptamers. J Colloid Interface Sci, 2007. 315(1): p. 99-106.

7. Kejik, P., et al., An integrated micro-Hall probe for scanning magnetic microscopy. Sensors and Actuators A, 2006. 129(1 -2): p. 212-215.

**Claims**

1.  A detection system for detecting a signal in a sandwich ELISA assay, the detection system comprising an amplification system and a sensing apparatus, the amplification system comprising:

    a binding molecule, said binding molecule being an antibody or an aptamer and said binding molecule being releasably attached to a first releasing molecule, wherein said first releasing molecule is a protease or a restriction enzyme;
    a cleaving agent, said cleaving agent being a protease or a restriction enzyme; and
    a plurality of molecular motors immobilised on a substrate, said molecular motors being selected from the group consisting of topoisomerases, polymerases (such as DNA polymerase), helicases, recombinases, chromatin and chromatin-remodelling factors, regulation factors, translocases and restriction-modification enzymes,

    and wherein the sensing apparatus comprises:

    a substrate;
    a polynucleotide attached at one end to the substrate;
    a magnetic bead attached to the other end of the polynucleotide;
    an element to produce a magnetic field; and
    an electronic sensor to detect movement of the magnetic bead.

2.  The system of claim 1, wherein a plurality of first releasing molecules are releasably attached to the binding molecule.

3.  The system of any preceding claim, wherein the binding molecule is releasably attached to the first releasing molecule by a linker.

4.  The system of claim 3, wherein the linker contains a cleavage site which is acted upon by the cleaving agent.

**5.** The system of any preceding claim, wherein the molecular motors are releasably attached to biotin.

**6.** The system of claim 5, wherein the molecular motors are attached to biotin by a linker.

**7.** The system of claim 6, wherein the linker contains a cleavage site which is recognised by the first releasing molecule.

**8.** The system of any preceding claim, wherein the electronic sensor is a magnetoresistive sensor or a Hall Effect sensor.

**9.** The system of any preceding claim, wherein the magnetic bead is a paramagnetic bead.

**10.** The system of any preceding claim, wherein the element to produce a magnetic field is an electromagnet.

**11.** The system of claim 10, wherein the electromagnet is a current carrying ring of wire.

**12.** The system of any preceding claim, further comprising a component to magnetise the magnetic bead.

**13.** The system of any preceding claim, wherein any electrical parts of the sensing apparatus are passivated.

**14.** The system of any preceding claim, wherein the apparatus is contained in a microfluidic chamber.

**15.** A detection method for detecting a signal in a sandwich ELISA assay, the method comprising:

exposing a cleaving agent, said cleaving agent being a protease or a restriction enzyme, to a first releasing molecule, said binding molecule being an antibody or an aptamer and said binding molecule being releasably attached to a binding molecule, said binding molecule being an antibody or an aptamer, to release the first releasing molecule from the binding molecule;
exposing the released first releasing molecule to a plurality of molecular motors, which have been immobilised on a substrate, to release a plurality of molecular motors from the substrate; and exposing the released molecular motors to a sensing apparatus to
detect the signal,

wherein the sensing apparatus comprises:

a surface;
a polynucleotide attached at one end to the surface;
a magnetic bead attached to the other end of the polynucleotide;
an element to produce a magnetic field; and
an electronic sensor to detect movement of the magnetic bead,
wherein the element produces a magnetic field which exerts a force on the magnetic bead to stretch the polynucleotide into an extended form,

wherein the electronic sensor detects movement of the magnetic bead resulting from movement of the polynucleotide;
wherein the molecular motor causes movement of the polynucleotide which causes the magnetic bead to move, this movement being detected by the electronic sensor; and
wherein said molecular motor is selected from the group consisting of topoisomerases, polymerases (such as DNA polymerase), helicases, recombinases, chromatin and chromatin-remodelling factors, regulation factors, translocases and restriction-modification enzymes.

**16.** The method of claim 16, further comprising the initial step of exposing the binding molecule, which is releasably attached to the first releasing molecule, to an antigen, or an antibody bound to an antigen.


**Patentansprüche**

**1.** Detektionssystem zur Detektion eines Signals in einem Sandwich-ELISA-Assay, wobei das Detektionssystem ein Amplifikationssystem und eine Sensorvorrichtung umfasst, wobei das Amplifikationssystem umfasst:

ein Bindungsmolekül, wobei das Bindungsmolekül ein Antikörper oder ein Aptamer ist, und wobei das Bindungsmolekül lösbar an ein erstes Freisetzungsmolekül gebunden ist, wobei das erste Freisetzungsmolekül eine Protease oder ein Restriktionsenzym ist;

ein Spaltungsagens, wobei das Spaltungsagens eine Protease oder ein Restriktionsenzym ist; und

eine Vielzahl von molekularen Motoren, die auf einem Substrat immobilisiert sind, wobei die molekularen Motoren aus der Gruppe bestehend aus Topoisomerasen, Polymerasen (wie beispielsweise DNA-Polymerase), Helikasen, Rekombinasen, Chromatin- und Chromatin-Remodellierungs-Faktoren, Regulationsfaktoren, Translokasen und Restriktions-Modifikations-Enzymen ausgewählt sind,

und wobei die Sensorvorrichtung umfasst:

ein Substrat;
ein Polynukleotid, welches an einem Ende an das Substrat gebunden ist;
ein magnetisches Kügelchen (Bead), welches an das andere Ende des Polynukleotids gebunden ist;

ein Element zur Erzeugung eines magnetischen Feldes; und
einen elektronischen Sensor zur Detektion der Bewegung des magnetischen Kügelchens.

2. System gemäß Anspruch 1, wobei eine Vielzahl an ersten Freisetzungsmolekülen lösbar an das Bindungsmolekül gebunden sind.

3. System gemäß irgendeinem vorhergehenden Anspruch, wobei das Bindungsmolekül über einen Linker lösbar an das erste Freisetzungsmolekül gebunden ist.

4. System gemäß Anspruch 3, wobei der Linker eine Spaltungsstelle enthält, auf die das Spaltungsagens einwirkt.

5. System gemäß irgendeinem vorhergehenden Anspruch, wobei die molekularen Motoren lösbar an Biotin gebunden sind.

6. System gemäß Anspruch 5, wobei die molekularen Motoren über einen Linker an Biotin gebunden sind.

7. System gemäß Anspruch 6, wobei der Linker eine Spaltungsstelle enthält, die durch das erste Freisetzungsmolekül erkannt wird.

8. System gemäß irgendeinem vorhergehenden Anspruch, wobei der elektronische Sensor ein magnetoresistiver Sensor oder ein Hall-Effekt-Sensor ist.

9. System gemäß irgendeinem vorhergehenden Anspruch, wobei das magnetische Kügelchen ein paramagnetisches Kügelchen ist.

10. System gemäß irgendeinem vorhergehenden Anspruch, wobei das Element zur Erzeugung eines magnetischen Feldes ein Elektromagnet ist.

11. System gemäß Anspruch 10, wobei der Elektromagnet ein stromführender Drahtring ist.

12. System gemäß irgendeinem vorhergehenden Anspruch, welches ferner eine Komponente zur Magnetisierung des magnetischen Kügelchens umfasst.

13. System gemäß irgendeinem vorhergehenden Anspruch, wobei sämtliche elektrische Teile der Sensorvorrichtung passiviert sind.

14. System gemäß irgendeinem vorhergehenden Anspruch, wobei die Vorrichtung in einer mikrofluidischen Kammer enthalten ist.

15. Detektionsverfahren zur Detektion eines Signals in einem Sandwich-ELISA-Assay, wobei das Verfahren umfasst:

Zusammenbringen eines Spaltungsagens, bei dem es sich um eine Protease oder ein Restriktionsenzym handelt, mit einem ersten Freisetzungsmolekül, wobei das Bindungsmolekül ein Antikörper oder ein Aptamer ist, und wobei das Bindungsmolekül lösbar an ein Bindungsmolekül gebunden ist, wobei das Bindungsmolekül ein Antikörper oder ein Aptamer ist, um das erste Freisetzungsmolekül von dem Bindungsmolekül abzulösen;

Zusammenbringen des freigesetzten ersten Freisetzungsmoleküls mit einer Vielzahl an molekularen Motoren, welche auf einem Substrat immobilisiert wurden, um eine Vielzahl an molekularen Motoren von dem Substrat abzulösen; und

Zusammenbringen der freigesetzten molekularen Motoren mit einer Sensorvorrichtung zur Detektion des Signals,

wobei die Sensorvorrichtung umfasst:

eine Oberfläche;

ein Polynukleotid, welches an einem Ende an die Oberfläche gebunden ist;

ein magnetisches Kügelchen (Bead), welches an das andere Ende des Polynukleotids gebunden ist;

ein Element zur Erzeugung eines magnetischen Feldes; und

einen elektronischen Sensor zur Detektion der Bewegung des magnetischen Kügelchens, wobei das Element ein magnetisches Feld erzeugt, welches eine Kraft auf das magnetische Kügelchen ausübt, um das Polynukleotid in eine verlängerte Form auszudehnen,

wobei der elektronische Sensor die Bewegung des magnetischen Kügelchens, die aus der Bewegung des Polynukleotids resultiert, detektiert;

wobei der molekulare Motor eine Bewegung des Polynukleotids verursacht, welche eine Bewegung des magnetischen Kügelchens bewirkt, wobei diese Bewegung durch den elektronischen Sensor detektiert wird; und

wobei der molekulare Motor aus der Gruppe bestehend aus Topoisomerasen, Polymerasen (wie beispielsweise DNA-Polymerase), Helikasen, Rekombinasen, Chromatin- und Chromatin-Remodellierungs-Faktoren, Regulationsfaktoren, Translokasen und Restriktions-Modifikations-Enzymen ausgewählt ist.

16. Verfahren gemäß Anspruch 16, welches ferner einen anfänglichen Schritt umfasst, in dem das Bindungsmolekül, welches lösbar an das erste Freisetzungsmolekül gebunden ist, mit einem Antigen oder einem an ein Antigen gebundenen Antikörper zusammengebracht wird.

## Revendications

1. Système de détection permettant de détecter un signal dans un essai ELISA en sandwich, le système de détection comprenant un système d'amplification et un appareil de détection, le système d'amplification comprenant :

une molécule de liaison, ladite molécule de liaison étant un anticorps ou un aptamère et ladite molécule de liaison étant fixée de façon détachable à une première molécule de libération, dans laquelle ladite première molécule de libération est une protéase ou une enzyme de restriction ;

un agent de clivage, ledit agent de clivage étant une protéase ou une enzyme de restriction ; et

une pluralité de moteurs moléculaires immobilisés sur un substrat, lesdits moteurs moléculaires étant sélectionnés dans le groupe consistant en les topoisomérases, les polymérases (telles que l'ADN polymérase), les hélicases, les recombinases, la chromatine et les facteurs de remodelage de la chromatine, les facteurs de régulation, les translocases et les enzymes de modification de restriction,

et dans lequel l'appareil de détection comprend :

un substrat ;

un polynucléotide fixé à une extrémité du substrat ;

une bille magnétique fixée à l'autre extrémité du polynucléotide ;

un élément servant à produire un champ magnétique ; et

un capteur électronique servant à détecter le mouvement de la bille magnétique.

2. Système selon la revendication 1, dans lequel une pluralité de premières molécules de libération est fixée de façon détachable à la molécule de liaison.

3. Système selon l'une quelconque des revendications précédentes, dans lequel la molécule de liaison est fixée de façon détachable à la première molécule de libération par un lieur.

4. Système selon la revendication 3, dans lequel le lieur contient un site de clivage sur lequel agit l'agent de clivage.

**5.** Système selon l'une quelconque des revendications précédentes, dans lequel les moteurs moléculaires sont fixés de façon détachable à la biotine.

**6.** Système selon la revendication 5, dans lequel les moteurs moléculaires sont fixés à la biotine par un lieur.

**7.** Système selon la revendication 6, dans lequel le lieur contient un site de clivage qui est reconnu par la première molécule de libération.

**8.** Système selon l'une quelconque des revendications précédentes, dans lequel le capteur électronique est un capteur magnétorésistif ou un capteur à effet Hall.

**9.** Système selon l'une quelconque des revendications précédentes, dans lequel la bille magnétique est une bille paramagnétique.

**10.** Système selon l'une quelconque des revendications précédentes, dans lequel l'élément servant à produire un champ magnétique est un électroaimant.

**11.** Système selon la revendication 10, dans lequel l'électroaimant est un anneau de fils portant un courant.

**12.** Système selon l'une quelconque des revendications précédentes, comprenant en outre un composant servant à aimanter la bille magnétique.

**13.** Système selon l'une quelconque des revendications précédentes, dans lequel toutes les parties électriques de l'appareil de détection sont passivées.

**14.** Système selon l'une quelconque des revendications précédentes, dans lequel l'appareil est contenu dans une chambre microfluidique.

**15.** Méthode de détection permettant de détecter un signal dans un essai ELISA en sandwich, la méthode comprenant :

l'exposition d'un agent de clivage, ledit agent de clivage étant une protéase ou une enzyme de restriction, à une première molécule de libération, ladite molécule de liaison étant un anticorps ou un aptamère et ladite molécule de liaison étant fixée de façon détachable à une molécule de liaison, ladite molécule de liaison étant un anticorps ou un aptamère, pour libérer la première molécule de libération de la molécule de liaison ; l'exposition de la première molécule de libération libérée à une pluralité de moteurs moléculaires, qui a été immobilisée sur un substrat, pour libérer une pluralité de moteurs moléculaires du substrat ; et l'exposition des moteurs moléculaires libérés à un appareil de détection pour détecter le signal,

dans laquelle l'appareil de détection comprend :

une surface ; un polynucléotide fixé à une extrémité de la surface ; une bille magnétique fixée à l'autre extrémité du polynucléotide ; un élément servant à produire un champ magnétique ; et un capteur électronique servant à détecter le mouvement de la bille magnétique, dans lequel l'élément produit un champ magnétique qui exerce une force sur la bille magnétique pour étirer le polynucléotide dans une forme étendue,

dans laquelle le capteur électronique détecte le mouvement de la bille magnétique résultant du mouvement du polynucléotide ; dans laquelle le moteur moléculaire entraînant un mouvement du polynucléotide, ce qui amène la bille magnétique à se déplacer, ce mouvement étant détecté par le capteur électronique ; et dans laquelle ledit moteur moléculaire est sélectionné dans le groupe consistant en les topoisomérases, les polymérases (telles que l'ADN polymérase), les hélicases, les recombinases, la chromatine et les facteurs de remodelage de la chromatine, les facteurs de régulation, les translocases et les enzymes de modification de restriction.

**16.** Méthode selon la revendication 16, comprenant en outre l'étape initiale d'exposition de la molécule de liaison, qui est fixée de façon détachable à la première molécule de libération, à un antigène ou à un anticorps lié à un antigène.

Figure 1

Figure 2

GST-( Pres.prot site)-HsdR
HsdR

Added PreScission™ Protease

GST

Figure 3

Release of GST (%)

EP 2 488 866 B1

Figure 4

Figure 5

Figure 6

Figure 7

Figure 8

Figure 9

Passivation

Substrate   Current Line

$H_{Bead}$

MR Sensor

3 μm   2.5 μm   1.5 μm

Magnetic Tweezers

Passivation

Substrate   Current Line

$H_{Bead}$

MR Sensor

EP 2 488 866 B1

Figure 10

$$\vec{F} = \vec{\nabla}(\vec{M}.\vec{B})$$

$$B_Z = \frac{\mu_o}{2} I_{Loop} \frac{R_{Loop}^2}{\left(Z^2 + R_{Loop}^2\right)^{3/2}}$$

Fz [pN]  Operation Point

Fz > 0.75 pN
@ 1 - 2.5 μm

$F_z$

$R_{Loop}$

Current Loop
$R_{Loop}$ = 6 μm
$I_{Loop}$ = 30 mA

$I_{Loop}$ [mA]

Fz [pN]
@ z = 2 μm

0.0

3.8

$R_{Loop}$ [μm]

Z [μm]

Figure 11a

Bead:
3 μm Micromer®
$\chi = 0.36$

$H_x = 1.36$ Oe

$H_x = 0.48$ Oe

$H_x = 0.27$ Oe

$I_{AC} = 55$ mA
$I_{DC} = 40$ mA

Separation [μm]

EP 2 488 866 B1

Figure 11b

**1 nm Vertical Displacement**

Bead:
3 μm Micromer®
$\chi = 0.36$

$I_{AC} = 55$ mA
$I_{MT} = 40$ mA

40 nT

y-axis: $|\Delta B_x [nT]|$ — 200, 150, 100, 50

x-axis: Separation [μm] — 2.0, 2.2, 2.4, 2.6, 2.8, 3.0, 3.2

EP 2 488 866 B1

Figure 13a

EP 2 488 866 B1

Figure 13b

EP 2 488 866 B1

## Figure 12

## Figure 14

| | Area [μm²] | V_Bias [mV] | S [%/Oe] |
|---|---|---|---|
| MgO MTJ | 20 x 1 | 25 | 11 |
| SF-SAF SV | 15 x 1 | 50 | 1.9 |

## Figure 15

Figure 16

Figure 17

$I_{MT}$ 0 mA    45 mA

Figure 18

Figure 19

Figure 20

**Figure 21**

**Figure 22**

$\Delta Hx = 55\ m\ Oe$

Base Line = 420 μV

$\Delta V = 1\ mV$

$(\Delta I_{MT} = 40\ mA)$

$\Delta Hx \sim 38\ Oe$

Base Line = 37.25 mV

Figure 23

**f = 34 kHz**
**I_AC = 15 mA**
**Amp = 0.6 Vpp**

**Strong Wash**

Figure 24

## Figure 25

## Figure 27

## Figure 28

## Figure 26

Figure 29

*a*

*b*

*c*

*d*

Figure 30

Figure 31

Figure 32

Figure 33

Figure 34

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0071681 A
- US 6528326 B
- US 20090135526 A
- GB 2351083 A
- GB 0918016 A
- US 61251562 B

### Non-patent literature cited in the description

- **PANDORI MW et al.** Photochemical control of the infectivity of adenoviral vectors using a novel photo-cleavable biotinylation reagent. *Chem. Biol.,* 2002, vol. 9 (5), 567-73
- **PETROTCHENKO EV et al.** BiPS, a photocleavable, isotopically coded, fluorescent cross-linker for structural proteomics. *Mol. Cell Proteomics,* February 2009, vol. 8 (2), 273-86
- **ELLINGTON AD ; SZOSTAK JW.** In vitro selection of RNA molecules that bind specific ligands. *Nature,* August 1990, vol. 346 (6287), 818-22
- **BOCK LC ; GRIFFIN LC ; LATHAM JA ; VERMAAS EH ; TOOLE JJ.** Selection of single-stranded DNA molecules that bind and inhibit human thrombin. *Nature,* February 1992, vol. 355 (6360), 564-6
- **HOPPE-SEYLER F ; BUTZ.** Peptide aptamers: powerful new tools for molecular medicine. *J Mol Med.,* 2000, vol. 78 (8), 426-30
- **HINCHEY ; MILLS.** Magnetic properties of superlattices formed from ferromagnetic and antiferromagnetic materials. *Phys. Rev. B,* 1986, vol. 33, 3329-3343
- **LEONG Z.Y. et al.** *Journal of Magnetism and Magnetic Materials,* March 2007, vol. 310 (2), e635-e637
- **KATO, K. et al.** Use of rabbit antibody IgG bound onto plain and aminoalkylsilyl glass surface for the enzyme-linked sandwich immunoassay. *J Biochem (Tokyo),* 1977, vol. 82 (1), 261-6
- **HUIZENGA, D.E. ; J.W. SZOSTAK.** A DNA aptamer that binds adenosine and ATP. *Biochemistry,* 1995, vol. 34 (2), 656-65
- **LAUHON, C.T. ; J.W. SZOSTAK.** RNA aptamers that bind flavin and nicotinamide redox cofactors. *J Am Chem Soc,* 1995, vol. 117 (4), 1246-57
- **BOCK, L.C. et al.** Selection of single-stranded DNA molecules that bind and inhibit human thrombin. *Nature,* 1992, vol. 355 (6360), 564-6
- **TANG, Q. ; X. SU ; K.P. LOH.** Surface plasmon resonance spectroscopy study of interfacial binding of thrombin to antithrombin DNA aptamers. *J Colloid Interface Sci,* 2007, vol. 315 (1), 99-106
- **KEJIK, P. et al.** An integrated micro-Hall probe for scanning magnetic microscopy. *Sensors and Actuators A,* 2006, vol. 129 (1 -2), 212-215